(19) Europäisches Patentamt
European Patent Office
Office européen des brevets

(11) EP 4 178 951 B1

(12) EUROPÄISCHE PATENTSCHRIFT

(45) Veröffentlichungstag und Bekanntmachung des Hinweises auf die Patenterteilung:
**11.12.2024 Patentblatt 2024/50**

(21) Anmeldenummer: **21752633.4**

(22) Anmeldetag: **09.07.2021**

(51) Internationale Patentklassifikation (IPC):
***C07D 277/46*** (2006.01) ***A61P 25/00*** (2006.01)
***C07B 59/00*** (2006.01) ***A61K 31/426*** (2006.01)

(52) Gemeinsame Patentklassifikation (CPC):
**C07D 277/46; A61P 25/00; C07B 59/002**

(86) Internationale Anmeldenummer:
**PCT/DE2021/100600**

(87) Internationale Veröffentlichungsnummer:
**WO 2022/008006 (13.01.2022 Gazette 2022/02)**

(54) **DEUTERIERTE UND TRITIIERTE N-(4,5-DIMETHYLTHIAZOL-2(3H)-YLIDEN)-2,2,3,3-TETRAMETHYLCYCLOPROPAN-1-CARBOXAMID-DERIVATE UND DEREN VERWENDUNG**

DEUTERATED AND TRITIATED N-(4,5-DIMETHYLTHIAZOLO-2(3H)-YLIDEN)-2,2,3,3-TETRAMETHYLCYCLOPROPANE-1-CARBOXAMIDE DERIVATIVES AND THEIR USE

DÉRIVÉS DE N-(4,5-DIMÉTHYLTHIAZOLO -2(3H)-YLIDÈNE) -2,2,3,3-TÉTRAMÉTHYLCYCLOPROPANE-1-CARBOXAMIDE DEUTÉRÉS ET TRITIÉS ET LEUR UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.07.2020 DE 102020118255**

(43) Veröffentlichungstag der Anmeldung:
**17.05.2023 Patentblatt 2023/20**

(73) Patentinhaber: **Helmholtz-Zentrum Dresden - Rossendorf e.V.**
**01328 Dresden (DE)**

(72) Erfinder:
- **MOLDOVAN, Rares-Petru**
  **01328 Dresden (DE)**
- **TEODORO, Rodrigo**
  **01328 Dresden (DE)**
- **TOUSSAINT, Magali**
  **01328 Dresden (DE)**
- **GÜNDEL, Daniel**
  **01328 Dresden (DE)**
- **DEUTHER-CONRAD, Winnie**
  **01328 Dresden (DE)**
- **BRUST, Peter**
  **01328 Dresden (DE)**

(74) Vertreter: **Riechelmann & Carlsohn Patentanwälte PartG mbB**
**Wiener Straße 91**
**01219 Dresden (DE)**

(56) Entgegenhaltungen:
- **RARES-PETRU MOLDOVAN ET AL: "Development of a High-Affinity PET Radioligand for Imaging Cannabinoid Subtype 2 Receptor", JOURNAL OF MEDICINAL CHEMISTRY, vol. 59, no. 17, 23 August 2016 (2016-08-23), pages 7840 - 7855, XP055622967, ISSN: 0022-2623, DOI: 10.1021/acs.jmedchem.6b00554**
- **FABIEN CAILLÉ ET AL: "From Structure-Activity Relationships on Thiazole Derivatives to the In Vivo Evaluation of a New Radiotracer for Cannabinoid Subtype 2 PET Imaging", MOLECULAR PHARMACEUTICS, vol. 14, no. 11, 18 October 2017 (2017-10-18), US, pages 4064 - 4078, XP055622952, ISSN: 1543-8384, DOI: 10.1021/acs.molpharmaceut.7b00746**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 4 178 951 B1

**Beschreibung**

Die Erfindung betrifft deuterierte und/oder tritiierte *N*-(4,5-Dimethylthiazol-2(3*H*)-yliden)-2,2,3,3-tetramethylcyclopropan-1-carboxamid-Derivate und deren Verwendung zur Herstellung radiofluorierter Verbindungen. Sie betrifft insbesondere Präkursoren für die Herstellung radiofluorierter deuterierter und tritiierter *N*-(4,5-Dimethylthiazol-2(3*H*)-yliden)-2,2,3,3-tetramethylcyclopropan-1-carboxamid-Derivate, die Verwendung der Präkursoren für diesen Zweck und radiofluorierte deuterierte und tritiierte *N*-(4,5-Dimethylthiazol-2(3*H*)-yliden)-2,2,3,3-tetramethylcyclopropan-1-carboxamid-Derivate. Bezugnahmen auf Methoden zur Behandlung des menschlichen oder tierischen Körpers in den folgenden Absätzen werden als Bezugnahmen auf Verbindungen zur Verwendung in Methoden zur Behandlung des menschlichen oder tierischen Körpers durch Therapie oder Diagnose interpretiert.

Aus WO 2009/067613 A1 sind Verbindungen der allgemeinen Formel S-1 bekannt,

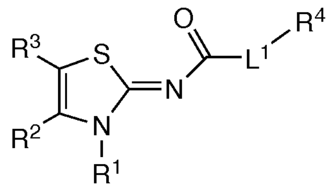

(Formel S-1),

die als Liganden für den Cannabinoid-Rezeptor 2 ($CNR_2$ oder $CB_2$) beschrieben werden. Die Verbindungen der allgemeinen Formel S-1 sollen als Therapeutika zur Behandlung von Krankheiten, die in Beziehung zu $CBR_2$ stehen, geeignet sein. Der Cannabinoid-Rezeptor 2 ist ein Bestandteil des Endocannabinoid-Systems und liegt überwiegend im Immunsystem vor. Der Rezeptor ist an der Regulation von Vorgängen im Körper beteiligt, insbesondere am Stoffwechsel und Immunfunktionen. Er vermittelt die Schmerzwahrnehmung und hat Einfluss auf die Neuronenaktivität. Die Beeinflussung des Cannabinoid-Rezeptors 2 kann eine Linderung von Schmerzen, insbesondere von chronischen Schmerzen, bewirken. Darüber hinaus vermittelt der Rezeptor die Wirkung von Cannabinoiden, wie (-)-$\Delta^9$-*trans*-Tetrahydrocannabinol, im Immunsystem.

Die aus WO 2009/067613 A1 bekannten Verbindungen sollen außerdem zur Behandlung von Entzündungskrankheiten, Immunkrankheiten, neurologischen Störungen, Krebserkrankungen des Immunsystems, Atemwegserkrankungen und kardiovaskulären Erkrankungen geeignet sein.

Ferner sind die nachstehend gezeigten radiomarkierten *N*-(4,5-Dimethylthiazol-2(3*H*)-yliden)-2,2,3,3-tetramethylcyclopropan-1-carboxamid-Derivate bekannt. Die Verbindung **[$^{11}$C]A836339** wurde von Horti et al. (Bioorg. Med. Chem. 2010, 14, 5202-5207) publiziert. Basierend auf dem (4,5-Dimethylthiazol-2(3*H*)-yliden)-2,2,3,3-tetra-methylcyclopropan-1-carboxamid-Grundgerüst wurden selektive, fluorierte $CBR_2$-Liganden von Moldovan et al. (J. Med. Chem. 2016, 59, 7840-7855 und J. Nuc. Med. 2015, 56, 53, 1048) synthetisiert und in einem Mausmodell für Neuroinflammation (LPS) untersucht. Zu diesen Verbindungen gehörte **[$^{18}$F]JHU94620.** Basierend auf Moldovan et al. (J. Med. Chem. 2016, 59, 7840-7855) wurde **[$^{18}$F]2f** von Caille et al. (Mol. Pharmaceutics 2017, 11, 4064-4078) entwickelt.

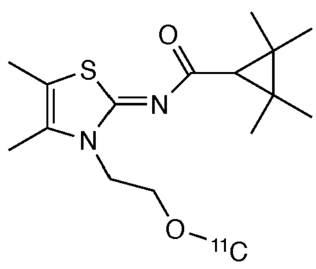

**[$^{11}$C]A836339**

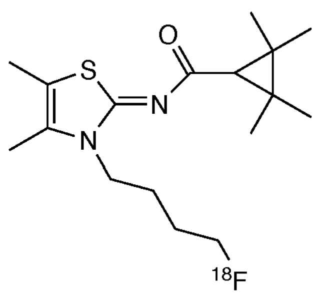

**[$^{18}$F]JHU94620**

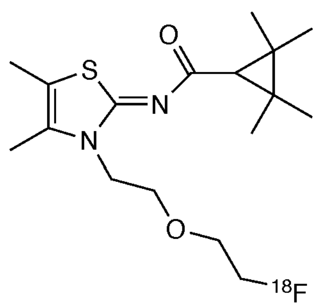

**[$^{18}$F]2f**

Die $^{18}$F-markierte Verbindung **[$^{18}$F]JHU94620** wird nach dem Stand der Technik (Moldovan et al. J. Med. Chem. 2016, 59, 7840-7855) über eine Radiosynthese hergestellt, die von einem Brom-Präkursor ausgeht. Der Brom-Präkursor entspricht dabei **[$^{18}$F]JHU94620,** außer dass sich an der Stelle des [$^{18}$F]-Atoms ein Bromatom als Abgangsgruppe befindet. **[$^{18}$F]JHU94620** weist jedoch eine geringe metabolische Stabilität auf.

Für eine medizinische Anwendung von Verbindung **[$^{18}$F]JHU94620** und anderen *N*-(4,5-Dimethylthiazol-2(3*H*-yliden)-2,2,3,3-tetramethylcyclopropan-1-carboxamid-Derivaten sollten die Verbindungen eine höhere metabolische Stabilität aufweisen.

Aufgabe der Erfindung ist es, die Nachteile nach dem Stand der Technik zu beseitigen. Es sollen insbesondere *N*-(4,5-Dimethylthiazol-2(3*H*)-yliden)-2,2,3,3-tetra-methylcyclopropan-1-carboxamid-Derivate angegeben werden, die nicht nur eine hohe Affinität und Selektivität gegenüber $CBR_2$, sondern auch eine hohe metabolische Stabilität besitzen und beispielsweise als Radiopharmaka oder Präkursoren für die Herstellung der Radiotracer verwendet werden können. Außerdem soll ein Verfahren zur Herstellung von *N*-(4,5-Dimethylthiazol-2(3*H*)-yliden)-2,2,3,3-tetramethylcyclopropan-1-carboxamid-Derivaten angegeben werden, das deren Herstellung mit hohen radiochemischen Ausbeuten ermöglicht.

Diese Aufgabe wird durch die Merkmale der Ansprüche 1, 8, 9, 11, 12 und 13 gelöst. Zweckmäßige Ausgestaltungen der Erfindungen ergeben sich aus den Merkmalen der Unteransprüche.

Nach Maßgabe der Erfindung ist eine Verbindung der allgemeinen Formel I vorgesehen:

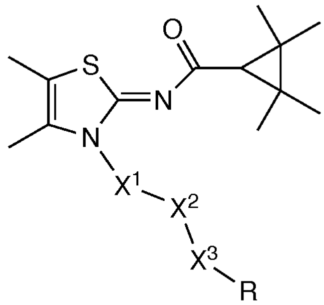

(Formel I),

worin

$X^1$ eine $CD_2$-Gruppe oder eine $CT_2$-Gruppe ist;
$X^2$ Sauerstoff oder eine Gruppe $(CZ^1Z^2)_n$ ist, wobei $Z^1$ und $Z^2$ bei jedem Auftreten unabhängig voneinander jeweils Wasserstoff, Deuterium oder Tritium sind und n eine Ganzzahl von 1 bis 12 ist;
$X^3$ eine $CD_2$-Gruppe oder eine $CT_2$-Gruppe ist; und
R aus der Gruppe ausgewählt ist, die aus Hydroxy, $-NO_2$, Halogen, einem Diazonium-Ion, einem Diazonium-Salz, einem Trialkylammonium-Ion, einem Trialkylammonium-Salz, einem Dialkoxyaren, einem Sulfoxid, einer Boronsäure, einem Boronsäureester, einer zinnorganischen Verbindung, einem Iodonium-Ion, einem Iodonium-Salz, einem Iodonium-Ylid und einem Sulfonat besteht.

Bei einer Verbindung der allgemeinen Formel I kann es sich um deren (*E*)-Isomer, deren (*Z*)-Isomer oder ein Gemisch aus dem (*E*)- und (*Z*)-Isomer handeln.

Es kann eine Verbindung der allgemeinen Formel I vorgesehen sein, worin

$X^1$ eine $CD_2$-Gruppe oder eine $CT_2$-Gruppe ist;
$X^2$ aus der Gruppe ausgewählt ist, die aus $(CH_2)_n$, $(CD_2)_n$, $(CT_2)_n$ und O besteht, wobei n eine Ganzzahl von 1 bis 12 ist;
$X^3$ eine $CD_2$-Gruppe oder eine $CT_2$-Gruppe ist; und
R aus der Gruppe ausgewählt ist, die aus Hydroxy, $-NO_2$, Halogen, einem Diazonium-Ion, einem Diazonium-Salz, einem Trialkylammonium-Ion, einem Trialkylammonium-Salz, einem Dialkoxyaren, einem Sulfoxid, einer Boronsäure, einem Boronsäureester, einer zinnorganischen Verbindung, einem Iodonium-Ion, einem Iodonium-Salz, einem Iodonium-Ylid und einem Sulfonat besteht.

Enthält zumindest einer der Reste $X^1$, $X^2$ und $X^3$ ein Deuteriumatom, so kann vorgesehen sein, dass die Reste $X^1$, $X^2$ und $X^3$ kein Tritiumatom enthalten. In diesem Fall ist die Verbindung der allgemeinen Formel I entweder eine deuterierte Verbindung oder eine tritiierte Verbindung, aber keine Verbindung, die - abgesehen von ihrem natürlichen Vorkommen - Deuterium und Tritium enthält. Bevorzugt sind die erfindungsgemäßen Verbindungen der allgemeinen Formel I deuterierte Verbindungen. Die deuterierten Verbindungen sind vorzugsweise keine tritiierten Verbindungen. Im Folgenden bezeichnet der Ausdruck "deuterierte Verbindung" eine Verbindung, die Deuteriumatome enthält, aber - abgesehen von ihrem natürlichen Vorkommen - keine Tritiumatome. Der Ausdruck "tritiierte Verbindung" bezeichnet hingegen eine Verbindung, die Tritiumatome enthält, aber - abgesehen von ihrem natürlichen Vorkommen - keine Deuteriumatome.

Es kann eine Verbindung der allgemeinen Formel I vorgesehen sein, worin

$X^1$ eine $CD_2$-Gruppe ist;
$X^2$ Sauerstoff oder eine Gruppe $(CZ^1Z^2)_n$ ist, wobei $Z^1$ und $Z^2$ bei jedem Auftreten unabhängig voneinander jeweils Wasserstoff oder Deuterium sind und n eine Ganzzahl von 1 bis 12 ist;
$X^3$ eine $CD_2$-Gruppe ist; und
R aus der Gruppe ausgewählt ist, die aus Hydroxy, $-NO_2$, Halogen, einem Diazonium-Ion, einem Diazonium-Salz, einem Trialkylammonium-Ion, einem Trialkylammonium-Salz, einem Dialkoxyaren, einem Sulfoxid, einer Boronsäure, einem Boronsäureester, einer zinnorganischen Verbindung, einem Iodonium-Ion, einem Iodonium-Salz, einem Iodonium-Ylid und einem Sulfonat besteht. Eine solche Verbindung ist eine deuterierte Verbindung.

Es kann eine Verbindung der allgemeinen Formel I vorgesehen sein, worin

$X^1$ eine $CD_2$-Gruppe ist;
$X^2$ aus der Gruppe ausgewählt ist, die aus $(CH_2)_n$, $(CD_2)_n$ und O besteht, wobei n eine Ganzzahl von 1 bis 12 ist;
$X^3$ eine $CD_2$-Gruppe ist; und
R aus der Gruppe ausgewählt ist, die aus Hydroxy, $-NO_2$, Halogen, einem Diazonium-Ion, einem Diazonium-Salz, einem Trialkylammonium-Ion, einem Trialkylammonium-Salz, einem Dialkoxyaren, einem Sulfoxid, einer Boronsäure, einem Boronsäureester, einer zinnorganischen Verbindung, einem Iodonium-Ion, einem Iodonium-Salz, einem Iodonium-Ylid und einem Sulfonat besteht. Eine solche Verbindung ist eine deuterierte Verbindung.

Es kann eine Verbindung der allgemeinen Formel I vorgesehen sein, worin

$X^1$ eine $CD_2$-Gruppe ist;
$X^2$ $(CH_2)_n$ oder $(CD_2)_n$ ist, wobei n eine Ganzzahl von 1 bis 12 ist;
$X^3$ eine $CD_2$-Gruppe ist; und
R aus der Gruppe ausgewählt ist, die aus Hydroxy, $-NO_2$, Halogen, einem Diazonium-Ion, einem Diazonium-Salz, einem Trialkylammonium-Ion, einem Trialkylammonium-Salz, einem Dialkoxyaren, einem Sulfoxid, einer Boronsäure, einem Boronsäureester, einer zinnorganischen Verbindung, einem Iodonium-Ion, einem Iodonium-Salz, einem Iodonium-Ylid und einem Sulfonat besteht. Eine solche Verbindung ist eine deuterierte Verbindung.

Erfindungsgemäß ist n eine Ganzzahl von 1 bis 12. Es kann vorgesehen sein, dass n 1, 2, 3, 4, 5 oder 6 ist. In einem Beispiel ist n = 2. In diesem Fall trägt das N-Atom der Dimethylthiazol-Gruppe eine *n*-Butyl-Gruppe.

Es kann vorgesehen sein, dass die Verbindung der allgemeinen Formel I eine Verbindung der allgemeinen Formel I-F oder eine Verbindung der allgemeinen Formel I-P ist:

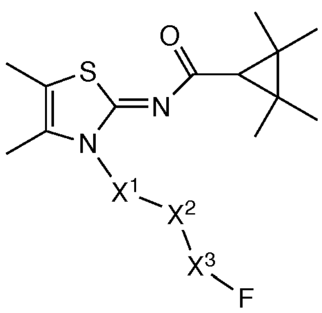

(Formel I-F)

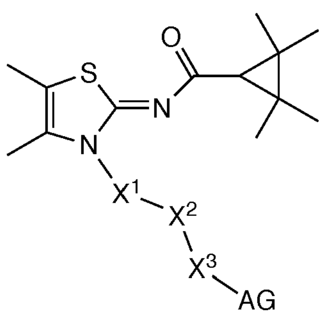

(Formel I-P)

worin $X^1$, $X^2$ und $X^3$ die im Zusammenhang mit Formel I angegebenen Bedeutungen haben. AG bezeichnet eine Abgangsgruppe, die aus der Gruppe ausgewählt ist, die aus Hydroxy, $-NO_2$, Chlor, Brom, Iod, einem Diazonium-Ion, einem Diazonium-Salz, einem Trialkylammonium-Ion, einem Trialkylammonium-Salz, einem Dialkoxyaren, einem Sulfoxid, einer Boronsäure, einem Boronsäureester, einer zinnorganischen Verbindung, einem Iodonium-Ion, einem Iodonium-Salz, einem Iodonium-Ylid und einem Sulfonat besteht. Bei einer Verbindung der allgemeinen Formel I-F kann es sich um deren (*E*)-Isomer, deren (*Z*)-Isomer oder ein Gemisch aus dem (*E*)- und (*Z*)-Isomer handeln. Bei einer Verbindung der allgemeinen Formel I-P kann es sich um deren (*E*)-Isomer, deren (*Z*)-Isomer oder ein Gemisch aus dem (*E*)- und (*Z*)-Isomer handeln.

Die Verbindung der Formel I-F ist eine Verbindung der allgemeinen Formel I, in der R Fluor ist. Die Verbindungen

der Formel I-F sind deuterierte und/oder tritiierte fluorierte Verbindungen. Es kann vorgesehen sein, dass die Verbindung der allgemeinen Formel I eine Verbindung der allgemeinen Formel [$^{18}$F]I-F ist:

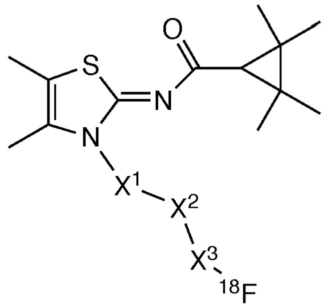

(Formel [$^{18}$F]I-F)

worin $X^1$, $X^2$ und $X^3$ die im Zusammenhang mit Formel I angegebenen Bedeutungen haben. Die Verbindung der Formel [$^{18}$F]I-F ist eine Verbindung der allgemeinen Formel I, in der R [$^{18}$F]Fluor ist. Die Verbindungen der Formel [$^{18}$F]I-F sind deuterierte und/oder tritiierte radiofluorierte Verbindungen. Bei einer Verbindung der allgemeinen Formel [$^{18}$F]I-F kann es sich um deren (*E*)-Isomer, deren (*Z*)-Isomer oder ein Gemisch aus dem (*E*)- und (*Z*)-Isomer handeln.

Die erfindungsgemäßen Verbindungen weisen eine hohe metabolische Stabilität auf. Das ist bei den Verbindungen der allgemeinen Formel I-F und [$^{18}$F]I-F besonders vorteilhaft, weil die in Positronen-Emissions-Tomographie (PET) eingesetzt werden sollen. Die hohe metabolische Stabilität verringert die Menge an Metaboliten im Gehirn (*in vivo*) stark. Der Einsatz von Verbindungen bei der PET-Bildgebung setzt voraus, dass nur geringe Mengen an Metaboliten in das Gehirn gelangen. Die erfindungsgemäßen Verbindungen der allgemeinen Formel I, insbesondere der allgemeinen Formel I-F, beispielsweise der Verbindungen der allgemeinen Formel [$^{18}$F]I-F, weisen eine hohe Bindungsaffinität und Selektivität gegenüber $CBR_2$ auf. Ihre Bindungsaffinität und Selektivität entsprechen denen der Verbindungen, die keine Deuterierung oder Tritiierung aufweisen. Sie können daher als hochaffine und selektive $CBR_2$-Radiopharmaka verwendet werden. Die erfindungsgemäßen $^{18}$F-markierten Radiotracer der allgemeinen Formel [$^{18}$F]I-F können zu *In-vitro*- und *In-vivo*-Untersuchungen der Expression und Verfügbarkeit von $CBR_2$ in Organismen durch geeignete Nachweisverfahren wie die Positronen-Emissions-Tomographie (PET) genutzt werden. Diese Untersuchungen ermöglichen es, genauere Kenntnisse über die Wirkung von Referenzverbindungen zu gewinnen und somit neben dem Radiopharmakon weitere Medikamente zu entwickeln und hinsichtlich ihrer potentiellen Wirksamkeit zu bewerten.

Die Verbindung der allgemeinen Formel I-P ist eine Verbindung der allgemeinen Formel I, wobei R eine Abgangsgruppe AG ist. Die Verbindungen der allgemeinen Formel I-P können insbesondere als Präkursoren für die Herstellung der Verbindungen der allgemeinen Formel [$^{18}$F]I-F verwendet werden.

Es kann vorgesehen sein, dass AG ein Sulfonat ist. Unter dem Begriff "Sulfonat" wird eine $R^S$-$SO_2$-O-Gruppe verstanden. $R^S$ kann beispielsweise eine verzweigte oder unverzweigte substituierte oder unsubstituierte $C_1$-$C_6$-Alkylgruppe, eine Arylgruppe oder eine Alkylarylgruppe sein. Vorzugsweise ist $R^S$ $CH_3$-, $CF_3$- oder $CH_3$-$C_6H_4$-. Das Sulfonat kann beispielsweise aus der Gruppe ausgewählt sein, die aus einer Toluolsulfonsäureester-Gruppe, einer Methylsulfonsäureester-Gruppe und einer Trifluormethylsulfonsäureester-Gruppe besteht. Unter einer Toluolsulfonsäureester-Gruppe wird eine -OTs-Gruppe verstanden, wobei Ts Tosyl ist. Unter einer Methylsulfonsäureester-Gruppe wird eine -OMs-Gruppe verstanden, wobei Ms Mesyl ist. Unter einer Trifluormethylsulfonsäureester-Gruppe wird $CF_3$-$SO_2$-O- verstanden. Vorzugsweise ist AG -OMs oder -OTs. Besonders bevorzugt ist AG -OTs.

Es kann vorgesehen sein, dass AG eine zinnorganische Verbindung ist. Dabei kann es sich um ein Zinnorganyl handeln. Bei der zinnorganischen Verbindung kann es sich beispielsweise um Alkylzinn, dessen Alkylgruppe(n) substituiert oder unsubstituiert sein können, wobei eine oder mehrere der Alkylgruppen gegebenenfalls ein oder mehrere Heteroatome aufweisen, oder Arylzinn, dessen Arylgruppe(n) substituiert oder unsubstituiert sein können, wobei eine oder mehrere der Arylgruppen gegebenenfalls ein oder mehrere Heteroatome aufweisen, handeln. Bei den Heteroatomen kann es sich beispielsweise um O, N, S oder P handeln.

Es kann vorgesehen sein, dass AG ein Halogen ist, das aus der Gruppe ausgewählt ist, die aus Chlor, Brom und Iod besteht.

Bevorzugte Beispiele von Verbindungen der allgemeinen Formel I sind in der nachstehenden Tabelle 1 angegeben. Bei den in Tabelle 1 angegebenen Verbindung kann es sich jeweils um deren (*E*)-Isomer, deren (*Z*)-Isomer oder ein Gemisch aus dem (*E*)- und (*Z*)-Isomer handeln.

Tabelle 1

| Verbindung | Struktur | Name |
|---|---|---|
| **[18F] JHU94620-D4** | 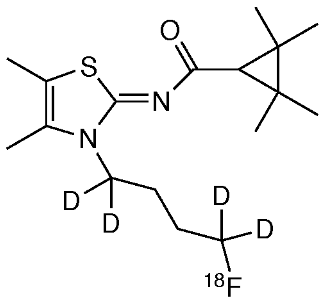 | *N*-(3-(4-([18F]Fluor)butyl-1,1,4,4-$d_4$)-4,5-dimethylthiazol-2(3*H*)-yliden)-2,2,3,3-tetramethylcyclopropan-1-carboxamid |
| **JHU94620-D4** | 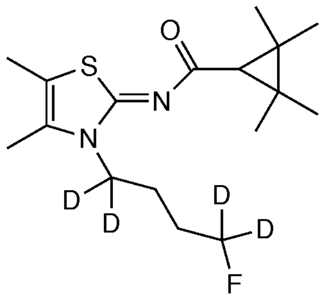 | *N*-(3-(4-Fluorbutyl-1,1,4,4-$d_4$)-4,5-dimethylthiazol-2(3*H*)-yliden)-2,2,3,3-tetra-methylcyclopropan-1-carboxamid |
| **3** | 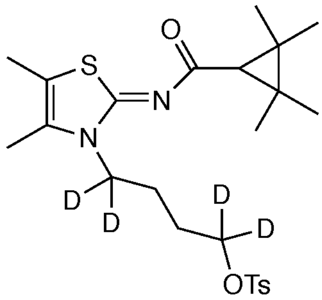 | 4-(4,5-Dimethyl-2-((2,2,3,3-tetramethylcyclopropan-1-carbonyl)-imino)thiazol-3(2*H*)-yl)butyl-1,1,4,4-$d_4$-4-methylbenzensulfonat |
| **[18F] JHU94620-D8** | 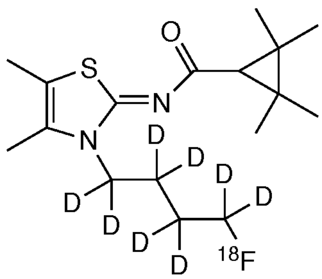 | *N*-(3-(4-([18F]Fluor)butyl-1,1,2,2,3,3,4,4-$d_8$)-4,5-dimethylthiazol-2(3*H*)-yli-den)-2,2,3,3-tetramethylcy-clopropan-1-carboxamid |
| **JHU94620-D8** | 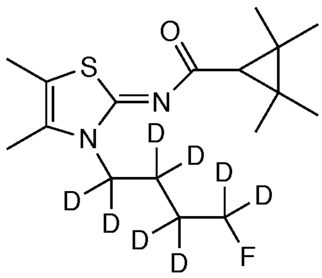 | *N*-(3-(4-Fluorbutyl-1,1,2,2,3,3,4,4-$d_8$)-4,5-dimethylthiazol-2(3*H*)-yliden)-2,2,3,3-tetramethylcy-clopropan-1-carboxamid |

(fortgesetzt)

| Verbindung | Struktur | Name |
| --- | --- | --- |
| **4** | 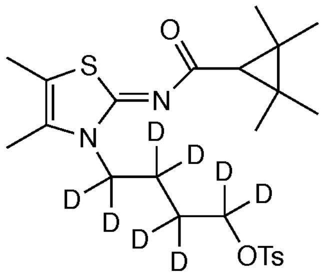 | 4-(4, 5-Dimethyl-2-((2,2,3,3-tetramethylcyclo-propan-1-carbonyl)imino)-thiazol-3(2*H*)-yl)butyl-1,1,2,2,3,3,4,4-$d_8$-4-methylbenzensulfonat |

Die Verbindungen **[$^{18}$F]JHU94620-D4** und **[$^{18}$F]JHU94620-D8** sind Verbindungen der allgemeinen Formel [$^{18}$F]I-F. Die Verbindungen **JHU94620-D4** und **JHU94620-D8** sind Verbindungen der allgemeinen Formel I-F. Die Verbindungen **3** und **4** sind Verbindungen der allgemeinen Formel I-P. Verbindung **3** kann insbesondere als Präkursor für die Herstellung von **[$^{18}$F]JHU94620-D4** eingesetzt werden. Verbindung **4** kann insbesondere als Präkursor für die Herstellung von **[$^{18}$F]JHU94620-D8** eingesetzt werden.

Es hat sich herausgestellt, dass die erfindungsgemäßen Verbindungen der allgemeinen Formel I, insbesondere der allgemeinen Formel I-F, Liganden für den Cannabinoid-Rezeptor 2 darstellen. Sie können daher für die Diagnostik und Therapie von Entzündungskrankheiten, Immunkrankheiten, neurologischen Störungen, Krebserkrankungen des Immunsystems, Atemwegserkrankungen und kardiovaskulären Erkrankungen genutzt werden. Sie können darüber hinaus zur Linderung von Schmerzen genutzt werden. Die erfindungsgemäßen Verbindungen der allgemeinen Formel I können somit als Medikament, insbesondere als Medikament für Entzündungskrankheiten, Immunkrankheiten, neurologische Störungen, Krebserkrankungen des Immunsystems, Atemwegserkrankungen und kardiovaskuläre Erkrankungen genutzt werden. Sie können darüber hinaus als Medikament zur Linderung von Schmerzen genutzt werden. Ebenso kann ein pharmazeutisch akzeptables Salz einer Verbindung der allgemeinen Formel I als Medikament, insbesondere als Medikament für Entzündungskrankheiten, Immunkrankheiten, neurologische Störungen, Krebserkrankungen des Immunsystems, Atemwegserkrankungen und kardiovaskuläre Erkrankungen oder als Medikament zur Linderung von Schmerzen genutzt werden. Entzündungskrankheiten umfassen beispielsweise Entzündungen von Nervengewebe (die auch als Neuroinflammation bezeichnet werden).

Nach Maßgabe der Erfindung ist ferner die Verwendung einer Verbindung der allgemeinen Formel I-P

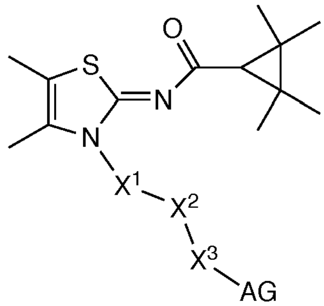

(Formel I-P)

zur Herstellung einer Verbindung der allgemeinen Formel [$^{18}$F]I-F

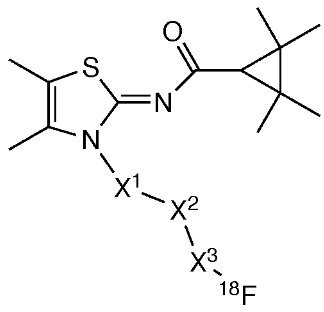

(Formel [$^{18}$F]I-F)

vorgesehen. Dabei entspricht die Verbindung der allgemeinen Formel I-P der Verbindung der allgemeinen Formel [$^{18}$F]I-F, außer dass die Abgangsgruppe AG durch [$^{18}$F]Fluor ersetzt ist. Alle anderen Substituenten, d. h. $X^1$, $X^2$ und $X^3$ einschließlich $Z^1$ und $Z^2$, sind unverändert und befinden sich in der gleichen Position. Die Substituenten $X^1$, $X^2$, $X^3$ und AG in den Formeln I-P und [$^{18}$F]I-F haben die vorstehend angegebenen Bedeutungen.

Die Verbindungen der allgemeinen Formel I-P sind besonders geeignete Präkursoren für die Herstellung von Verbindungen der allgemeinen Formel [$^{18}$F]I-F. Beispielsweise kann Verbindung **3** als Präkursor für die Herstellung von **[$^{18}$F]JHU94620-D4** eingesetzt werden.

Nach Maßgabe der Erfindung ist außerdem ein Verfahren zur Herstellung einer Verbindung der allgemeinen Formel I-F, z. B. einer Verbindung der allgemeinen Formel [$^{18}$F]I-F, vorgesehen. Das Verfahren umfasst die Schritte:

(a) Umsetzen einer Verbindung der Formel 11

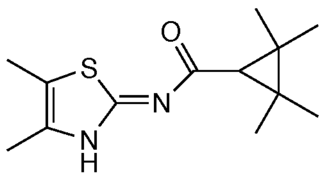

(Formel 11)

mit einer Verbindung der allgemeinen Formel II

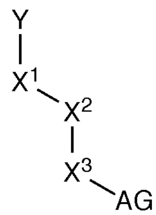

(Formel II)

worin $X^1$, $X^2$, $X^3$ und AG die im Zusammenhang mit der allgemeinen Formel I-P angegebenen Bedeutung haben und Y ein Sulfonat ist, zu einer Verbindung der allgemeinen Formel I-P; und

(b) Umsetzen der Verbindung der allgemeinen Formel I-P zu der Verbindung der allgemeinen Formel I-F mit einem Fluorierungsmittel.

Bei der Verbindung der Formel 11 kann es sich um deren (*E*)-Isomer, deren (*Z*)-Isomer oder ein Gemisch aus dem (*E*)- und (*Z*)-Isomer handeln.

In der Verbindung der allgemeinen Formel II ist Y vorzugsweise Y -O-Ts oder O-Ms, besonders bevorzugt O-Ts, wobei Ts eine Tosylgruppe und Ms eine Mesylgruppe bezeichnet. Es kann ferner vorgesehen sein, dass AG ebenfalls ein Sulfonat ist. In diesem Fall können beispielsweise Y und AG jeweils dieselbe Bedeutung aufweisen. So kann in einer bevorzugten Ausführungsform sowohl Y als auch AG jeweils -O-Ts sein. Ein Beispiel einer Verbindung der allgemeinen

Formel II ist Butan-1,4-diyl-1,1,4,4-$d_4$-bis(4-methylbenzensulfonat) (**12**). Ein anderes Beispiel einer Verbindung der allgemeinen Formel II ist Butan-1,4-diyl-1,1,2,2,3,3,4,4-$d_8$-bis(4-methylbenzensulfonat) (**13**).

Die in Schritt (a) des erfindungsgemäßen Verfahrens vorgesehene Umsetzung von Verbindung 11 mit einer Verbindung der allgemeinen Formel II kann in einem organischen Lösungsmittel durchgeführt werden. Bei dem organischen Lösungsmittel kann es sich um ein nicht-protisches, polares Lösungsmittel wie Acetonitril (MeCN), Dimethylformamid (DMF), *N,N*-Dimethylacetamid (DMAA), N-Methyl-2-pyrrolidon (NMP), Dimethylsulfoxid (DMSO) oder Gemische davon handeln. Vorzugsweise wird Dimethylformamid als Lösungsmittel verwendet. Die Temperatur liegt bevorzugt zwischen 0 und 100 °C, stärker bevorzugt zwischen 20 und 100 °C, noch stärker bevorzugt zwischen 40 und 80 °C, besonders bevorzugt bei 60 °C. Die Umsetzung wird vorzugsweise bei Raumtemperatur oder einer erhöhten Temperatur durchgeführt. Bevorzugt wird die Umsetzung für einen Zeitraum von 10 min bis 2 h, stärker bevorzugt von 30 min bis 1,5 h und besonders bevorzugt 60 min durchgeführt. Die Umsetzung wird vorzugsweise bei Umgebungsdruck durchgeführt. Die Umsetzung wird vorzugsweise unter Bewegung des Reaktionsgemisches, beispielsweise unter Rühren, durchgeführt. Verbindung 11 und die Verbindung der allgemeinen Formel II liegen vorzugsweise in einem molaren Verhältnis von 1 : 2 bis 2 : 1, bevorzugt von 1 : 1 vor. Die Umsetzung kann in Gegenwart eines Hydrids, bevorzugt eines Alkali- oder Erdalkalimetallhydrids durchgeführt werden. Ein bevorzugtes Hydrid ist Natriumhydrid (NaH). Die Umsetzung kann unter einer Schutzgasatmosphäre, beispielsweise unter einer Stickstoff- oder Argonatmosphäre vorgenommen werden. Die Konzentration von Verbindung 11, der Verbindung der allgemeinen Formel II und, falls vorgesehen, des Hydrids in dem Lösungsmittel, sollte - bezogen auf die Lösung - jeweils wenigstens 10 Gew.-% betragen.

Schritt (b) sieht das Umsetzen der Verbindung der allgemeinen Formel I-P zu der Verbindung der allgemeinen Formel I-F mit einem Fluorierungsmittel vor. Die Auswahl des Fluorierungsmittels und der Reaktionsbedingungen kann davon abhängen, ob die Verbindung der allgemeinen Formel I-F eine Verbindung der allgemeinen Formel [$^{18}$F]I-F ist oder nicht radiofluoriert ist.

<u>Schritt (b): Umsetzung zu einer Verbindung der allgemeinen Formel [$^{18}$F]I-F</u>

Handelt es sich bei der Verbindung der allgemeinen Formel I-F um eine Verbindung der allgemeinen Formel [$^{18}$F]I-F, so kann es sich bei dem Fluorierungsmittel um [$^{18}$F]Fluorid-Anionen handeln. Die [$^{18}$F]Fluorid-Anionen können in einer Lösung vorliegen, die im Folgenden als [$^{18}$F]Fluorid-haltige Lösung bezeichnet wird. In Schritt (b) kann zur Herstellung der [$^{18}$F]Fluorid-haltigen Lösung das [$^{18}$F]Fluorid-Anion mittels bekannter Verfahren hergestellt werden. Beispielsweise wird das [$^{18}$F]Fluorid-Anion im Zyklotron durch Bestrahlung von mindestens 97%-ig angereichertem $H_2{}^{18}O$ mit Protonen einer Energie von 9,6 MeV hergestellt. Die auf diesem Wege erhaltene wässrige [$^{18}$F]Fluorid-haltige Lösung kann auf einer Anionentauscherkartusche (QMA) fixiert und mit Hilfe einer wässrigen Lösung einer Base, wie Kaliumcarbonat, Cäsiumcarbonat, Natriumhydrid oder Tetraalkylammoniumhydrogencarbonat eluiert werden. Vorzugsweise wird eine wässrige Lösung von Kaliumcarbonat als Base verwendet. Die Elution der basischen [$^{18}$F]Fluorid-haltigen Lösung erfolgt in ein Reaktionsgefäß, das einen Phasen-Transfer-Katalysator (PTC), wie Kronenether, quartäre Ammoniumsalze oder Alkali- oder Erdalkalisalze, enthält. Als PTC werden vorzugsweise ein [2,2,2]-Kryptand (Kryptofix® oder K222), Tetra-n-Butyl-Ammonium-phosphat, -hydroxid, -oxalat, Toluensulfonat, oder wahlweise andere Kronenether wie 18-Krone-6 genutzt. Der auf diese Weise erhaltene [$^{18}$F]Fluorid-Komplex kann einer azeotropen Trocknung unter Vakuum unterzogen werden. Bei dem organischen Lösungsmittel kann es sich um ein nicht-protisches, polares Lösungsmittel wie Acetonitril (MeCN), Dimethylformamid (DMF), *N,N*-Dimethylacetamid (DMAA), N-Methyl-2-pyrrolidon (NMP), Dimethylsulfoxid (DMSO) oder Gemische davon handeln. Vorzugsweise wird Acetonitril als Lösungsmittel verwendet. Die azeotrope Trocknung wird vorzugsweise unter thermischer Reaktionsführung im geschlossenen Reaktionsgefäß bei erhöhter Temperatur durchgeführt. Die Temperatur liegt vorzugsweise zwischen 50 und 60 °C. Die azeotrope Trocknung kann auch mit Unterstützung von Mikrowellen durchgeführt werden. Dazu können Mikrowellen mit einer Leistung von 50 bis 150 W, bevorzugt 65 bis 85 W und besonders bevorzugt 75 W verwendet werden.

Zur Durchführung von Schritt (b) wird der azeotrop getrocknete [$^{18}$F]Fluorid-Komplex vorzugsweise in einem organischen Lösungsmittel gelöst. Auf diese Weise wird die [$^{18}$F]Fluorid-haltige Lösung erhalten. Bei dem organischen Lösungsmittel kann es sich um ein nicht-protisches, polares Lösungsmittel wie Acetonitril (MeCN), Dimethylformamid (DMF), *N,N*-Dimethylacetamid (DMAA), N-Methyl-2-pyrrolidon (NMP), Dimethylsulfoxid (DMSO) oder Gemische davon handeln. Vorzugsweise wird Acetonitril als Lösungsmittel verwendet. Zu dieser Lösung wird dann der Präkursor der allgemeinen Formel P-1 gegeben. Vorzugsweise wird der Präkursor zuvor in einem organischen Lösungsmittel gelöst. Bei diesem Lösungsmittel handelt es sich vorzugsweise um das gleiche Lösungsmittel, das als Lösungsmittel in der [$^{18}$F]Fluorid-haltigen Lösung enthalten ist, also um Acetonitril. Der in dem Lösungsmittel gelöste Präkursor wird dann zu der [$^{18}$F]Fluorid-haltigen Lösung gegeben. Schritt (b) des erfindungsgemäßen Verfahrens wird vorzugsweise bei einer erhöhten Temperatur durchgeführt. Die Temperatur liegt vorzugsweise zwischen 80 und 110 °C, besonders bevorzugt bei 90 °C. Vorzugsweise wird der Schritt (b) für einen Zeitraum von 5 bis 15 min und besonders bevorzugt 10 min durchgeführt. Schritt (b) des erfindungsgemäßen Verfahrens wird vorzugsweise bei Umgebungsdruck durchgeführt. Schritt (b) wird vorzugsweise unter Bewegung des Reaktionsgemisches, beispielsweise unter Rühren, durchgeführt.

Schritt (b): Umsetzung zu einer Verbindung der allgemeinen Formel 1-F, die kein [18F]-Fluor enthält

Handelt es sich bei der Verbindung der allgemeinen Formel I-F nicht um eine Verbindung der allgemeinen Formel [18F]I-F, so kann es sich bei dem Fluorierungsmittel um eine Verbindung handeln, die eine nukleophile Fluorierung der Verbindung der allgemeinen Formel I-P ermöglicht. Beispielsweise kann das Fluorierungsmittel ein Tetraalkylammoniumfluorid sein. Das Tetraalkylammoniumfluorid kann aus der Gruppe ausgewählt sein, die aus Tetrabutylammoniumfluorid (TBAF), Tetraethylammoniumfluorid (TEAF) und Tetramethylammoniumfluorid (TMAF) besteht.

Schritt (b) kann in einem organischen Lösungsmittel durchgeführt werden. Bei dem organischen Lösungsmittel kann es sich um ein nicht-protisches, polares Lösungsmittel wie Tetrahydrofuran (THF), Acetonitril (MeCN), Dimethylformamid (DMF), *N*,*N*-Dimethylacetamid (DMAA), N-Methyl-2-pyrrolidon (NMP), Dimethylsulfoxid (DMSO) oder Gemische davon handeln. Vorzugsweise wird Tetrahydrofuran als Lösungsmittel verwendet. Die Temperatur liegt bevorzugt zwischen 0 und 100 °C, stärker bevorzugt zwischen 20 und 80 °C, noch stärker bevorzugt zwischen 40 und 60 °C, besonders bevorzugt bei 60 °C. Die Umsetzung wird vorzugsweise bei Raumtemperatur oder einer erhöhten Temperatur durchgeführt. Bevorzugt wird die Umsetzung für einen Zeitraum von 10 min bis 2 h, stärker bevorzugt von 30 min bis 1,5 h und besonders bevorzugt 60 min durchgeführt. Die Umsetzung wird vorzugsweise bei Umgebungsdruck durchgeführt. Die Umsetzung wird vorzugsweise unter Bewegung des Reaktionsgemisches, beispielsweise unter Rühren, durchgeführt. Die Verbindung der allgemeinen Formel I-P und das Fluorierungsmittel liegen vorzugsweise in einem molaren Verhältnis von 1 : 3 bis 2 : 1, bevorzugt von 2 : 1 vor. Die Umsetzung kann unter einer Schutzgasatmosphäre, beispielsweise unter einer Stickstoff- oder Argonatmosphäre vorgenommen werden. Die Konzentration der Verbindung der allgemeinen Formel I-P und des Fluorierungsmittels, sollte - bezogen auf die Lösung - jeweils wenigstens 10 Gew.-% betragen.

Schema 1 veranschaulicht die Herstellung einer Verbindung der allgemeinen Formel [18F]I-F gemäß dem erfindungsgemäßen Verfahren:

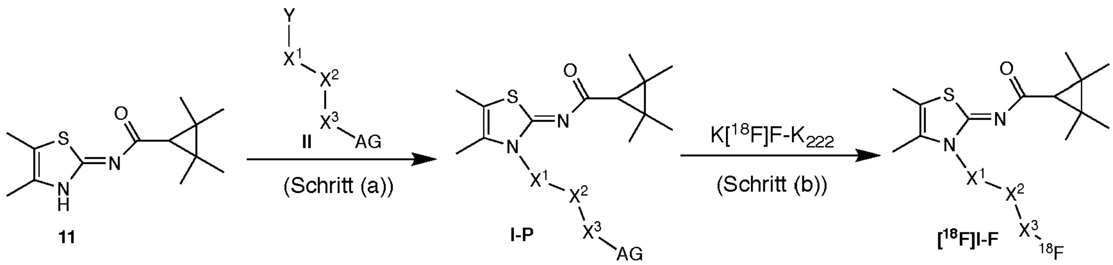


Schema 1

Schema 2 veranschaulicht die Herstellung einer Verbindung der allgemeinen Formel I-F, die kein [18F]Fluor enthält, gemäß dem erfindungsgemäßen Verfahren:

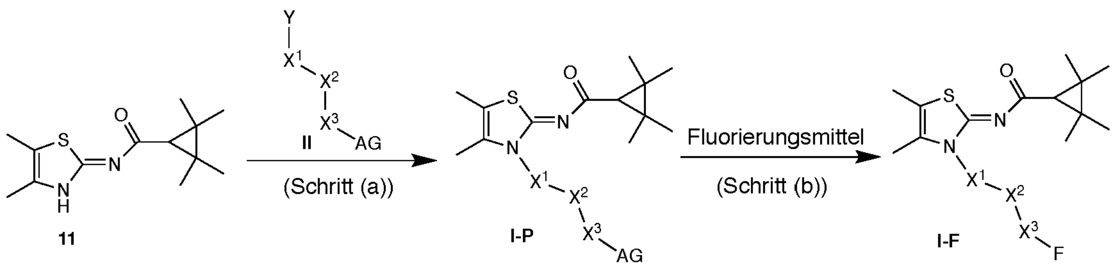


Schema 2

Schritt (a) ermöglicht die Herstellung der Verbindungen der allgemeinen Formel I-P. Schritt (a) ist somit ein Verfahren zur Herstellung einer Verbindung der allgemeinen Formel I-P. Schema 3 veranschaulicht die Herstellung einer Verbindung der allgemeinen Formel I-P:

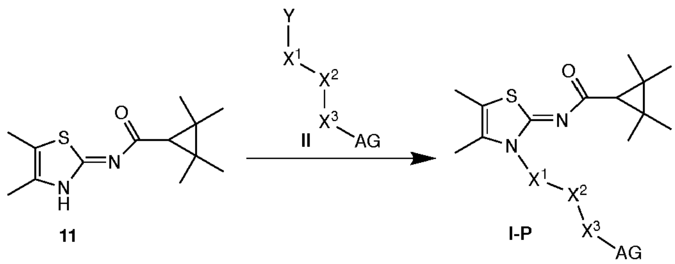


Schema 3

Die im Zusammenhang mit Schritt (a) beschriebene Verfahrensweise kann auch zur Herstellung einer Verbindung der allgemeinen Formel I-F verwendet werden, mit dem Unterschied, dass anstelle von einer Verbindung der allgemeinen Formel II eine Verbindung der allgemeinen Formel III

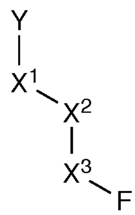

(Formel III)

verwendet wird. In der Verbindung der allgemeinen Formel III haben $X^1$, $X^2$ und $X^3$ die im Zusammenhang mit der allgemeinen Formel I-F angegebenen Bedeutungen. Schema 4 veranschaulicht die Herstellung einer Verbindung der allgemeinen Formel I-F. Dabei kann vorgesehen sein, dass die Verbindung III kein [$^{18}$F]Fluor enthält.

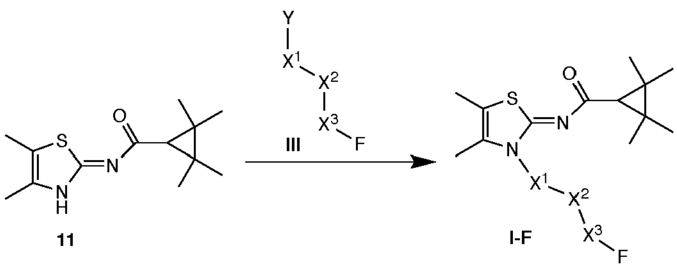


Schema 4

Die Erfindung ermöglicht insbesondere die Radiosynthese von deuterierten Verbindungen der allgemeinen Formel [$^{18}$F]I-F. Insbesondere die deuterierten Verbindungen der allgemeinen Formel [$^{18}$F]I-F können als Radiotracer für die nuklearmedizinische Bildgebung von $CBR_2$ mittels Positronen-Emissions-Tomographie (PET) verwendet werden. Die deuterierten Verbindungen der allgemeinen Formel [$^{18}$F]I-F weisen eine hohe Affinität und Selektivität gegenüber $CBR_2$ auf.

Es ist bekannt, dass Wasserstoff ein Mischelement ist. Bei der Herstellung der aus dem Stand der Technik bekannten Verbindung **[$^{18}$F]JHU94620** können daher geringe Menge an deuterierten und/oder tritiierten Isotopologen erhalten werden. Der Anteil der deuterierten und/oder tritiierten Isotopologe ist allerdings gering, weil er durch die natürliche Häufigkeit von Deuterium und Tritium bestimmt wird. In der vorliegenden Erfindung ist jedes Atom, das nicht ausdrücklich als spezielles Isotop benannt wird, ein stabiles Isotop. Sofern nichts anderes angegeben ist, wird unter einem Rest, der als Wasserstoff oder H bezeichnet ist, ein Rest verstanden, der Wasserstoff in seinem natürlichen Isotopenverhältnis aufweist. Sofern nichts anderes angegeben ist, wird unter einem Rest, der als Deuterium oder D bezeichnet ist, ein Rest verstanden, der Deuterium in einer Häufigkeit aufweist, die zumindest 3000 mal größer ist als die natürliche Häufigkeit von Deuterium. Unter Annahme einer natürlichen Häufigkeit von Deuterium von 0,015 % bedeutet eine 3000 mal höhere Häufigkeit einen Deuteriumeinbau von 45 %. Sofern nichts anderes angegeben ist, wird unter einem Rest, der als Tritium oder T bezeichnet ist, ein Rest verstanden, der Tritium in einer Häufigkeit aufweist,

die zumindest 4,5*$10^{16}$ mal größer ist als die natürliche Häufigkeit von Tritium. Unter Annahme einer natürlichen Häufigkeit von Tritium von $10^{-15}$ % bedeutet eine 4,5*$10^{16}$ mal höhere Häufigkeit einen Tritiumeinbau von 45 %. Der Ausdruck "Isotopologe" bezeichnet Moleküle, die sich nur in ihrer Isotopenzusammensetzung unterscheiden. Sie haben die gleiche chemische Formel und die gleichen Bindungsverhältnisse zwischen den Atomen, unterscheiden sich aber durch zumindest ein Atom, das eine andere Zahl an Neutronen aufweist.

Das Verhältnis zwischen der Häufigkeit eines Isotops in einer Verbindung und der natürlichen Häufigkeit des Isotops wird als "Isotopen-Anreicherungsfaktor" bezeichnet. Der Isotopen-Anreicherungsfaktor soll, wie vorstehend beschrieben, für jedes als Deuterium bezeichnete Atom zumindest 3000 (45 % Einbau von Deuterium bei einem als Deuterium bezeichneten Rest) betragen. Der Isotopen-Anreicherungsfaktor kann zumindest 3500 (52,5 % Einbau von Deuterium), zumindest 4000 (60 % Einbau von Deuterium), zumindest 4500 (67,5 % Einbau von Deuterium), zumindest 5000 (75 % Einbau von Deuterium), zumindest 5500 (82,5 % Einbau von Deuterium), zumindest 6000 (90 % Einbau von Deuterium), zumindest 6333,3 (95 % Einbau von Deuterium), zumindest 6466,7 (97 % Einbau von Deuterium), zumindest 6600 (99 % Einbau von Deuterium) oder zumindest 6633,3 (99,5 % Einbau von Deuterium) betragen. Der Isotopen-Anreicherungsfaktor soll, wie vorstehend beschrieben, für jedes als Tritium bezeichnete Atom zumindest 4,5*$10^{16}$ (45 % Einbau von Tritium bei einem als Tritium bezeichneten Rest) betragen. Der Isotopen-Anreicherungsfaktor kann zumindest 5,25*$10^{16}$ (52,5 % Einbau von Tritium), zumindest 6 *$10^{16}$ (60 % Einbau von Tritium), zumindest 6,75*$10^{16}$ (67,5 % Einbau von Tritium), zumindest 7,5*$10^{16}$ (75 % Einbau von Tritium), zumindest 8,25*$10^{16}$ (82,5 % Einbau von Tritium), zumindest 9*$10^{16}$ (90 % Einbau von Tritium), zumindest 9,5*$10^{16}$,3 (95 % Einbau von Tritium), zumindest 9,7*$10^{16}$ (97 % Einbau von Tritium), zumindest 9,9*$10^{16}$ (99 % Einbau von Tritium) oder zumindest 9,95*$10^{16}$ (99,5 % Einbau von Tritium) betragen.

Eine Verbindung, die nach Maßgabe der Erfindung zumindest ein Deuteriumatom oder ein Tritiumatom aufweisen soll, kann als Gruppe von Isotopologen angesehen werden. Der Anteil der Isotopologen, die eine Verbindung bilden, kann variieren. Eine Verbindung, die nach Maßgabe der Erfindung zumindest ein Deuteriumatom oder ein Tritiumatom aufweisen soll, enthält geringere Mengen an Isotopologen, die Wasserstoffatome anstelle eines oder mehrerer der angegebenen Deuteriumatome oder Tritiumatome aufweisen. Die relative Menge dieser Isotopologen sollte, bezogen auf die Verbindung, weniger als 55 % der Verbindung betragen. Es kann vorgesehen sein, dass die relative Menge dieser Isotopologen weniger als 50 %, weniger als 47,5 %, weniger als 40 %, weniger als 32,5 %, weniger als 25 %, weniger als 17,5 %, weniger als 10 %, weniger als 5 %, weniger als 3 %, weniger als 1 % oder weniger als 0,5 % beträgt.

Der Ausdruck "Alkyl" bezieht sich, sofern nichts anderes angegeben ist, insbesondere auf eine gesättigte aliphatische Kohlenwasserstoff-Gruppe mit einer verzweigten oder unverzweigten Kohlenstoffkette mit 1 bis 12 Kohlenstoffatomen, vorzugsweise 1 bis 8 Kohlenstoffatomen und besonders bevorzugt 1 bis 6 Kohlenstoffatomen. Beispiele von Alkylgruppen umfassen, sind aber nicht beschränkt auf Methyl, Ethyl, Propyl, Isopropyl, Isobutyl, *sec*-Butyl, *tert*-Butyl, Pentyl, *n*-Hexyl, Octyl, Dodecyl und dergleichen. Die Alkylgruppe kann gegebenenfalls mit einem oder mehreren Substituenten substituiert sein, wobei jeder Substituent unabhängig Hydroxy, Alkyl, Alkoxy, Halogen, Halogenalkyl, Amino, Monoalkylamino oder Dialkylamino ist, wenn nicht speziell anders angegeben.

Der Ausdruck "Alkoxy" bezieht sich, sofern nichts anderes angegeben ist, insbesondere auf eine Gruppe der Formel -OR, worin R eine Alkylgruppe ist, wie hierin definiert. Beispiele von Alkoxykomponenten umfassen, sind aber nicht beschränkt auf Methoxy, Ethoxy, Isopropoxy und dergleichen. Die Alkoxygruppe kann gegebenenfalls mit einem oder mehreren Substituenten substituiert sein, wobei jeder Substituent unabhängig Hydroxy, Alkyl, Alkoxy, Halogen, Halogenalkyl, Amino, Monoalkylamino oder Dialkylamino ist, wenn nicht speziell anders angegeben.

Der Ausdruck "Aryl" oder "Aren" bezieht sich, sofern nichts anderes angegeben ist, auf eine cyclische, aromatische Kohlenwasserstoffgruppe, die aus einem mono-, bi- oder tricyclischen aromatischen Ringsystem mit 5 bis 15 Ringatomen, bevorzugt 5 oder 6 Ringatomen, besteht. Die Arylgruppe kann gegebenenfalls eine substituierte Arylgruppe sein. Beispiele von Arylgruppen umfassen, sind aber nicht beschränkt auf Phenyl, Naphthyl, Anthracenyl, Naphthalenyl, Phenanthryl, Fluorenyl, Indenyl, Pentalenyl, Azulenyl, Oxydiphenyl, Biphenyl, Methylendiphenyl, Aminodiphenyl, Diphenylsulfidyl, Diphenylsulfonyl, Diphenylisopropylidenyl, Benzodioxanyl, Benzofuranyl, Benzodioxylyl, Benzopyranyl, Benzoxazinyl, Benzoxazinonyl, Benzopiperadinyl, Benzopiperazinyl, Benzopyrrolidinyl, Benzomorpholinyl, Methylendioxyphenyl, Ethylendioxyphenyl und dergleichen, einschließlich teilweise hydrierte Derivate davon. Ein bevorzugtes Beispiel ist Phenyl. Der Ausdruck "substituierte Arylgruppe" bezieht sich insbesondere auf eine Arylgruppe, die gegebenenfalls unabhängig mit ein bis vier Substituenten, bevorzugt einem oder zwei Substituenten, ausgewählt aus Alkyl, Cycloalkyl, Heteroalkyl, Hydroxyalkyl, Halogen, Nitro, Cyano, Hydroxy, Alkoxy, Amino, Acylamino, Monoalkylamino, Dialkylamino, Halogenalkyl, Halogenalkoxy, Harnstoff, Amido, Alkansulfonyl, -COR (worin R Wasserstoff, Alkyl, Phenyl oder Phenylalkyl ist), $-(CR'R'')_n-COOR$ (worin n eine Ganzzahl von 0 bis 5 ist, R' und R" unabhängig voneinander Wasserstoff oder Alkyl sind, und R Wasserstoff, Alkyl, Cycloalkyl, Cycloalkylalkyl, Phenyl oder Phenylalkyl ist) oder $-(CR'R'')_n-CONR^{a'}R^{b'}$ (worin n eine Ganzzahl von 0 bis 5 ist, R' und R" unabhängig voneinander Wasserstoff oder Alkyl sind und $R^{a'}$ und $R^{b'}$ unabhängig voneinander Wasserstoff, Alkyl, Cycloalkyl, Cycloalkylalkyl, Phenyl oder Phenylalkyl sind), substituiert ist. Beispiele eines Arens umfassen, sind aber nicht beschränkt auf Benzen, Naphthalin und Anthracen.

Der Ausdruck "Acyl" bezieht sich, sofern nichts anderes angegeben ist, insbesondere auf eine Gruppe der

Formel -C(=O)R, wobei R Wasserstoff oder Alkyl, wie hierin definiert, ist.

Der Ausdruck "Halogen" bezieht sich, sofern nichts anderes angegeben ist, auf Fluor, Chlor, Brom oder Iod.

Die Erfindung wird nachstehend anhand von Ausführungsbeispielen, die die Erfindung nicht einschränken sollen, unter Bezugnahme auf die Zeichnungen näher erläutert. Dabei zeigen

Fig. 1 ein UV-HPLC-Chromatogramm der Referenzverbindung **JHU94620-D4;**

Fig. 2 ein Radio-HPLC-Chromatogramm der erfindungsgemäßen Verbindung **[$^{18}$F]JHU94620-D4;**

Fig. 3 ein Radio-HPLC-Chromatogramm von Hirnhomogenat 30 p.i. von **[$^{18}$F]JHU94620;** und

Fig. 4 ein Radio-HPLC-Chromatogramm von Hirnhomogenat 30 p.i. von **[$^{18}$F]JHU94620-D4**

Beispiel 1

Synthese von *N*-(3-(4-([$^{18}$F]Fluor)butyl-1,1,4,4-$d_4$)-4,5-dimethylthiazol-2(3*H*)-yliden)-2,2,3,3-tetramethylcyclopropan-1-carboxamid (**[$^{18}$F]JHU94620-D4**)

Schema 5 zeigt die Herstellung der erfindungsgemäßen Verbindung **[$^{18}$F]JHU94620-D4** durch Umsetzung von *N*-(4,5-Dimethylthiazol-2(3H)-yliden)-2,2,3,3-tetramethylcyclopropan-1-carboxamid (**11**) mit Butan-1,4-diyl-1,1,4,4-$d_4$-bis(4-methylbenzensulfonat) (**12**) zum Präkursor (**3**) und die anschließende Umsetzung von Verbindung **3** zu **[$^{18}$F] JHU94620-D4**

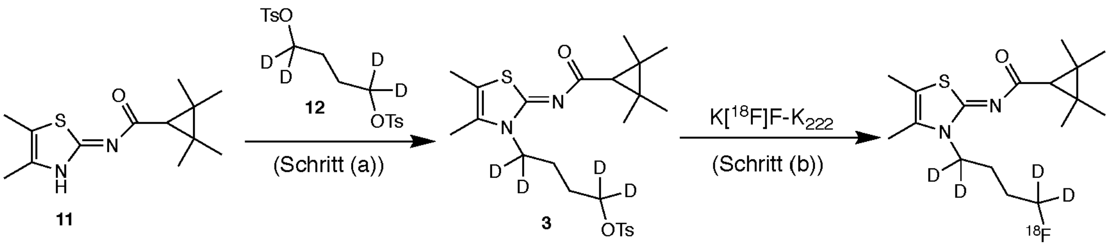

11
3
[$^{18}$F]JHU94620-D4

Schema 5

Bei Verbindung **12** handelt es sich um eine Verbindung der allgemeinen Formel II, in der $X^1$ $CD_2$ ist, $X^2$ $(CH_2)_2$ ist, $X^3$ $CD_2$ ist, Y -OTs ist und AG -OTs ist. K[$^{18}$F]F-$K_{222}$ bezeichnet den [$^{18}$F]$F^-$/$K_{222}$/$K^+$-Komplex, dessen Herstellung nachstehend beschrieben ist.

Schritt (a): Synthese von 4-(4,5-Dimethyl-2-((2,2,3,3-tetramethylcyclopropan-1-carbonyl)-imino)thiazol-3(2*H*)-yl)butyl-1,1,4,4-$d_4$-4-methylbenzensulfonat (**3**)

Zu einer Lösung von Verbindung **11** (1 eq, 0,6 mmol) und Verbindung 12 (1,5 eq, 0,9 mmol) in 3 ml DMF wurde NaH (60 %, 2 eq, 1,2 mmol) gegeben und die Mischung 1 h auf 60 °C unter Argonatmosphäre erhitzt. Anschließend wurde das Lösungsmittel am Rotationsverdampfer entfernt. Der Rückstand wurde in Ethylacetat (EA) aufgenommen (10 ml) und mit einer wässrigen 5%igen $NaHCO_3$-Lösung (10 ml) und anschließend mit gesättigter NaCl-Lösung (100 ml) gewaschen. Trocknung über $MgSO_4$ und Entfernen des Lösungsmittels ergaben ein gelbes Öl, welches mittels Säulenchromatographie (Kieselgel, EA : Petrolether (PE) von 1/20 bis 1/4) gereinigt wurde. Verbindung **3** wurde als weißer Feststoff mit einer Ausbeute von 33 % erhalten.

$^1$H-NMR (400 MHz, $CDCl_3$) $\delta$ 7,81 (d, *J* = 8,3 Hz, 2H), 7,36 (d, *J* = 8,0 Hz, 2H), 2,47 (s, 3H), 2,16 (s, 6H), 1,75 (m, 4H) 1,50 (s, 1H), 1,33 (s, 6H), 1,21 (s, 6H). HRMS (ESI+): *m/z* (%) = 483,2278, ber. 483,2284 für $C_{24}H_{31}D_4FN_2O_4S_2^+$ $[M+H]^+$.

Schritt (b): Synthese von *N*-(3-(4-([$^{18}$F]Fluor)butyl-1,1,4,4-$d_4$)-4,5-dimethylthiazol-2(3*H*)-yliden)-2,2,3,3-tetramethylcyclopropan-1-carboxamid (**[$^{18}$F]JHU94620-D4**)

Das nach der Bestrahlung erhaltene wässrige [$^{18}$F]Fluorid (2 bis 3 GBq) wurde zu 1 ml Wasser gegeben, auf einer Anionenaustauscherkartusche (QMA) fixiert und mit einer wässrigen $K_2CO_3$-Lösung (1,8 mg in 300 ml Wasser)

in eine Lösung aus 1 ml MeCN und Kryptofix ($K_{222}$, 5,6 mg) eluiert. Die azeotrope Trocknung des Komplexes erfolgte mikrowellenunterstützt (Power cycling, 75 W, 50 - 60 °C, Argonstrom) unter Vakuum. Der gebildete $[^{18}F]F^{-}K_{222}/K^{+}$-Komplex wurde mit 2 mg des Präkursors **3** (in 600 ml MeCN) versetzt. Anschließend wurde das Reaktionsgemisch bei 90 °C für 10 min gerührt.

Zur Bestimmung der Markierungsausbeute wurde ein Aliquot entnommen und mit Radio-DC (49 ± 4 %, n = 3) und Radio-HPLC (48 ± 5 %, n = 2) untersucht. Die Reinigung und Isolation des Radiotracers erfolgte mittels semipräparativer RP-HPLC (Säule: ReproSil-Pur 120 C18-AQ, 250 x 20 mm, 5 mm; Eluent: 65 % MeCN/20 mM $NH_4OAc_{aq.}$; Fluss: 4,2 ml/min). Die gesammelte Produktfraktion wurde mit Wasser verdünnt (20 ml), auf einer Sep-Pak®-C18-Plus-Kartusche sorbiert und mit Ethanol (EtOH) (1,0 ml) eluiert. Anschließend wurde das Lösungsmittel unter Erwärmen im Argonstrom entfernt und in einer 0,9%-igen Kochsalzlösung formuliert (≤ 10 % Ethanol, v/v). Innerhalb einer Synthesezeit von ca. 102 min wurde das Produkt **[$^{18}$F]JHU94620-D4** isoliert und durch Radio-HPLC und Radio-DC untersucht, wobei die Identität des Produktes durch Co-Injektion der Referenzverbindung bestätigt wurde (siehe Figuren 1 und 2). Die radiochemische Reinheit war ≥ 99 %. Die radiochemische Ausbeute betrug etwa 20 bis 25 %. Die molare Aktivität betrug 200 ± 20 GBq/μmol.

Vergleichsbeispiel 1

Synthese von *N*-(3-(4-([$^{18}$F]Fluor)butyl)-4,5-dimethylthiazol-2(3*H*)-yliden)-2,2,3,3-tetramethylcyclopropan-1-carboxamid (**[$^{18}$F]JHU94620**)

Zu Vergleichszwecken wurde **[$^{18}$F]JHU94620,** wie in Moldovan et al. J. Med. Chem. 2016, 59, 7840-7855 beschrieben, unter Verwendung eines Brom-Präkursors hergestellt. **[$^{18}$F]JHU94620** wurde mit einer radiochemischen Ausbeute von 16 % und einer molaren Aktivität von 170 GBq/μmol erhalten.

Beispiel 2

Synthese von *N*-(3-(4-Fluorbutyl-1,1,4,4-*d*$_4$)-4,5-dimethylthiazol-2(3*H*)-yliden)-2,2,3,3-tetramethylcyclopropan-1-carboxamid (**JHU94620-D4**)

Schema 6 zeigt die Herstellung der erfindungsgemäßen Verbindung **JHU94620-D4** durch Umsetzung von *N*-(4,5-Dimethylthiazol-2(3H)-yliden)-2,2,3,3-tetramethylcyclopropan-1-carboxamid (**11**) mit Butan-1,4-diyl-1,1,4,4-*d*$_4$-bis(4-methylbenzensulfonat) (**12**) zum Präkursor (**3**) und die anschließende Umsetzung von Verbindung **3** zu **JHU94620-D4.**

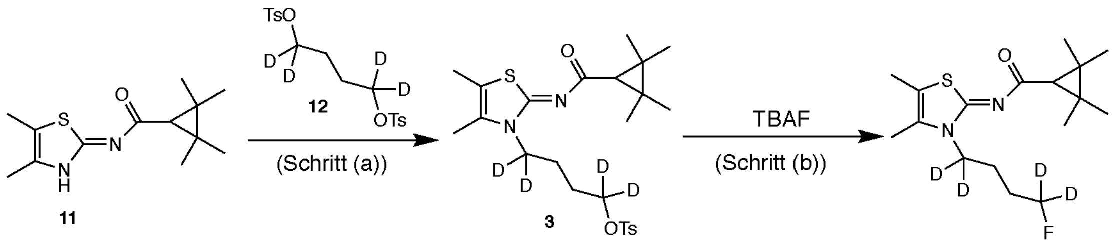


JHU94620-D4

Schema 6

TBAF bezeichnet Tetrabutylammoniumfluorid.

Schritt (a): Synthese von 4-(4,5-Dimethyl-2-((2,2,3,3-tetramethylcyclopropan-1-carbonyl)-imino)thiazol-3(2*H*)-yl)butyl-1,1,4,4-*d*$_4$-4-methylbenzensulfonat (**3**)

Schritt (a) des in Schema 6 gezeigten Verfahrens wurde wie in Beispiel 1, Schritt (a) beschrieben durchgeführt.

Schritt (b): Synthese von *N*-(3-(4-Fluorbutyl-1,1,4,4-*d*$_4$)-4,5-dimethylthiazol-2(3*H*)-y-liden)-2,2,3,3-tetramethylcyclopropan-1-carboxamid (**JHU94620-D4**)

Zu einer Lösung von Verbindung **3** (1 eq, 0,5 mmol) in 3 ml THF wurde TBAF (1M im THF, 2 eq, 1 mmol) gegeben und die Mischung 1 h auf 50 °C unter Argonatmosphäre erhitzt. Anschließend wurde das Lösungsmittel am

Rotationsverdampfer entfernt. Der Rückstand wurde in Ethylacetat (EA) aufgenommen (10 ml) und mit einer wässrigen 5%igen $NaHCO_3$-Lösung (10 ml) und anschließend mit gesättigter NaCl-Lösung (100 ml) gewaschen. Trocknung über $MgSO_4$ und Entfernen des Lösungsmittels ergaben ein gelbes Öl, welches mittels Säulenchromatographie (Kieselgel, EA : Petrolether (PE) von 1/20 bis 1/4) gereinigt wurde. Verbindung **JHU94620-D4** wurde als weißer Feststoff mit einer Ausbeute von 72 % erhalten.

$^1$H-NMR (300 MHz, $CDCl_3$) δ 2,20 (s, 3H), 2,18 (s, 3H), 1,55 (s, 1H), 1,36 (s, 6H), 1,24 (s, 6H). HRMS (ESI+): *m*/*z* (%) = 331,2152, ber. 331,2152 für $C_{17}H_{24}D_4FN_2OS^+$ [M+H]$^+$.

Beispiel 3

Synthese von *N*-(3-(4-([$^{18}$F]Fluor)butyl-1,1,2,2,3,3,4,4-*d*$_8$)-4,5-dimethylthiazol-2(3*H*)-yliden)-2,2,3,3-tetramethylcyclopropan-1-carboxamid (**[$^{18}$F]JHU94620-D8**)

Schema 7 zeigt die Herstellung der erfindungsgemäßen Verbindung **[$^{18}$F]JHU94620-D8** durch Umsetzung von *N*-(4,5-Dimethylthiazol-2(3*H*)-yliden)-2,2,3,3-tetramethylcyclopropan-1-carboxamid (**11**) mit Butan-1,4-diyl-1,1,2,2,3,3,4,4-*d*$_8$-bis(4-methylbenzensulfonat) (**13**) zum Präkursor (**4**) und die anschließende Umsetzung von Verbindung **4** zu **[$^{18}$F]JHU94620-D8.**

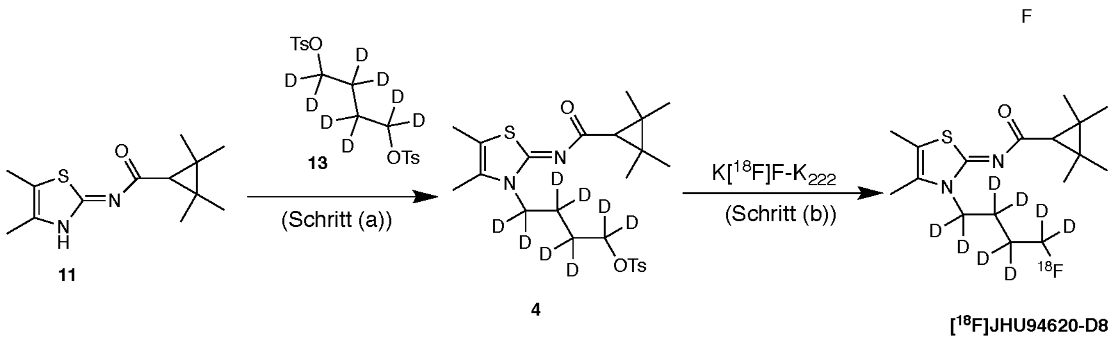

13
11
4
[$^{18}$F]JHU94620-D8

Schema 7

Bei Verbindung **13** handelt es sich um eine Verbindung der allgemeinen Formel II, in der X$^1$ $CD_2$ ist, X$^2$ $(CD_2)_2$ ist, X$^3$ $CD_2$ ist, Y -OTs ist und AG -OTs ist.

Schritt (a): Synthese von 4-(4.5-Dimethyl-2-((2,2,3,3-tetramethylcyclopropan-1-carbonyl)-imino)thiazol-3(2*H*)-yl)butyl-1,1,2,2,3,3,4,4-*d*$_8$-4-methylbenzensulfonat (**4**)

Verbindung **4** wurde in der gleichen Weise wie in Beispiel 1, Schritt (a) hergestellt, außer das anstelle von Verbindung **12** Verbindung **13** eingesetzt wurde.

$^1$H-NMR (400 MHz, $CDCl_3$) δ 7,81 (d, *J* = 8,3 Hz, 2H), 7,36 (d, *J* = 8,0 Hz, 2H), 2,47 (s, 3H), 2,16 (s, 6H), 1,50 (s, 1H), 1,33 (s, 6H), 1,21 (s, 6H). HRMS (ESI+): *m*/*z* (%) = 487,2537, ber. 487,2535 für $C_{24}H_{27}D_8N_2O_4S_2^+$ [M+H]$^+$.

Schritt (b): Synthese von *N*-(3-(4-([$^{18}$F]Fluor)butyl-1,1,2,2,3,3,4,4-*d*$_8$)-4,5-dimethylthiazol-2(3*H*)-yliden)-2,2,3,3-tetramethylcyclopropan-1-carboxamid (**[$^{18}$F]JHU94620-D8)**

**[$^{18}$F]JHU94620-D8** wurde in der gleichen Weise wie in Beispiel 1, Schritt (b) hergestellt, außer das anstelle von Verbindung **3** Verbindung **4** eingesetzt wurde.

Beispiel 4

Synthese von *N*-(3-(4-Fluorbutyl-1,1,2,2,3,3,4,4-*d*$_8$)-4,5-dimethylthiazol-2(3*H*)-yli-den)-2,2,3,3-tetramethylcyclopropan-1-carboxamid (**JHU94620-D8**)

Schema 8 zeigt die Herstellung der erfindungsgemäßen Verbindung **JHU94620-D8** durch Umsetzung von *N*-(4,5-Dimethylthiazol-2(3H)-yliden)-2,2,3,3-tetramethylcyclopropan-1-carboxamid (**11**) mit Butan-1,4-diyl-1,1,2,2,3,3,4,4-*d*$_8$-bis(4-methylbenzensulfonat) (**13**) zum Präkursor (**4**) und die anschließende Umsetzung von Verbindung **4** zu **JHU94620-D8.**

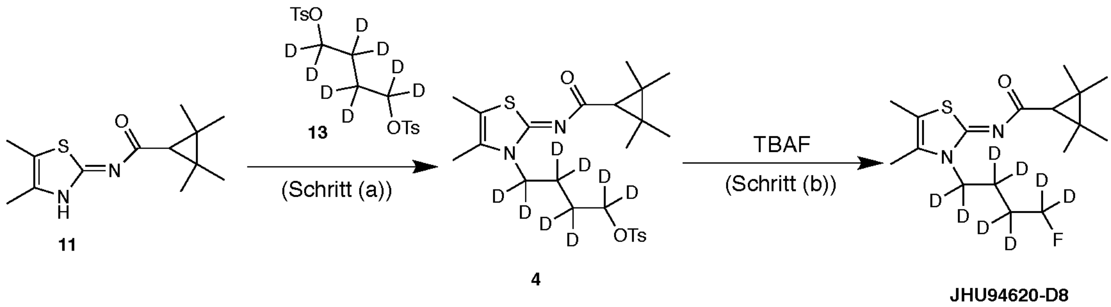


11 13 4 JHU94620-D8

Schema 8

Schritt (a): Synthese von 4-(4,5-Dimethyl-2-((2,2,3,3-tetramethylcyclopropan-1-carbonyl)-imino)thiazol-3(2*H*)-yl)butyl-1,1,2,2,3,3,4,4-$d_8$-4-methylbenzensulfonat (**4**)

Schritt (a) des in Schema 8 gezeigten Verfahrens wurde wie in Beispiel 1, Schritt (a) beschrieben durchgeführt.

Schritt (b): Synthese von *N*-(3-(4-Fluorbuiyl-1,1,2,2,3,3,4,4-$d_8$)-4,5-dimethylthiazol-2(3*H*)-yliden)-2,2,3,3-tetramethylcyclopropan-1-carboxamid (**JHU94620-D8**)

**JHU94620-D8** wurde in der gleichen Weise wie in Beispiel 2, Schritt (b) hergestellt, außer dass anstelle von Verbindung **3** Verbindung **4** eingesetzt wurde.

$^1$H-NMR (300 MHz, $CDCl_3$) $\delta$ 2,20 (s, 3H), 2,18 (s, 3H), 1,93 - 1,81 (m, 2H), 1,77 - 1,68 (m, 2H), 1,55 (s, 1H), 1,36 (s, 6H), 1,24 (s, 6H). HRMS (ESI+): *m/z* (%) = 335,2402, ber. 335,2403 für $C_{17}H_{20}D_8FN_2OS^+$ $[M+H]^+$.

Beispiel 5

Untersuchung der metabolischen Stabilität von **[$^{18}$F]JHU94620-D4**

Die metabolische Stabilität von **[$^{18}$F]JHU94620-D4** wurde gemäß dem von Moldovan et al., J. Med. Chem. 2016, 59, 7840-7855, beschriebenen Verfahren bestimmt. In gleicher Weise wurde die metabolische Stabilität von **JHU94620** und **[$^{18}$F]JHU94620** bestimmt. Die in Figuren 3 und 4 gezeigten Radio-HPLC-Chromatogramme wurde unter Verwendung einer ReproSil-Pur-C18-AQ-Säule 250 x 4,6 mm, 5 mm; Eluent: 10 - 90 - 10 % MeCN / 20 mM $NH_4OAc$; Flow: 1 ml/min) bestimmt. **JHU94620** weist folgende Formel auf:

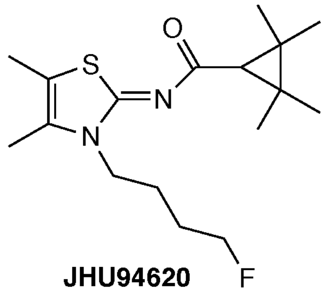

**JHU94620**

**JHU94620** ist das nicht-radiofluorierte und nicht-deuterierte Analogon zu **[$^{18}$F]JHU94620-D4** und **[$^{18}$F] JHU94620-D8.**

Es ist in Fig. 4 zu erkennen, dass in der *In-vivo*-Metabolitenstudie in weiblichen CD-1 Mäusen 30 min nach Injektion der Anteil von intaktem Radiotracer **[$^{18}$F]JHU94620-D4** im Gehirn ~80 % (Radio-HPLC, Extraktionsausbeute >97 %) und im Plasma ~15 % (Radio-HPLC, Extraktionsausbeute >95 %) betrug. Fig. 3 zeigt, dass der Anteil an intaktem Radiotracer **[$^{18}$F]JHU94620** nur 38 % betrug. **[$^{18}$F]JHU94620-D4** weist somit eine wesentlich höhere metabolische Stabilität als **[$^{18}$F]JHU94620** auf.

In der *In-vivo*-Metabolitenstudie in weiblichen CD-1-Mäusen betrug bei **JΣIU94620** 30 min nach Injektion der Anteil von intaktem Radiotracer im Gehirn 36 % (Radio-HPLC, n = 3, Extraktionsausbeute 93 %) und im Plasma 7 % (Radio-HPLC, n = 3, Extraktionsausbeute 94 %) der Gesamtaktivität.

Die metabolische Stabilität von **[$^{18}$F]JHU94620-D8** ähnelt der von **[$^{18}$F]JHU94620-D4** in hohem Maße.

Beispiel 6

Bestimmung der $CBR_2$-Gleichgewichts-Dissoziationskonstante $K_D$ von **[$^{18}$F]JHU94620-D8**

Die Gleichgewichts-Dissoziationskonstante $K_D$ wurde mittels eines homologen Kompetitionsassays bestimmt. Dazu wurde eine konstante Menge an Bindungsprotein (Homogenat von stabil mit humanem $CBR_2$-Rezeptor transfizierten CHO-Zellen; 2 Mio. Zellen/ml Ansatz) mit einer konstanten Menge an **[$^{18}$F]JHU94620-D8** (0,101 nM im Ansatz) und einer zunehmenden Konzentration von **JHU94620** (0,01 nM - 10 $\mu$M im Ansatz) für 60 Minuten bei Raumtemperatur in Bindungspuffer (50 mM TRIS-HCl, pH 7,4, mit 5 mM $MgCl_2$, 1 mM EDTA, 1 % BSA) inkubiert. Die Abtrennung von rezeptorgebundenem und freiem Radioliganden erfolgte durch Filtration über einen mit 0,3 % Polyethylenimin inkubierten GF/B-Glasfaserfilter. Die Quantifizierung von rezeptorgebundenem Radioliganden erfolgte durch Messung der filtergebundenen Radioaktivität in einem Gammacounter. Die Bestimmung der unspezifischen Bindung von **[$^{18}$F]JHU94620-D8** erfolgte durch Ko-Inkubation mit CP55940 (10 $\mu$M im Ansatz), die Bestimmung der Adsorption des Radioliganden an den Glasfaserfilter durch Inkubation ohne Bindungsprotein. Aus dem in diesem Experiment erhaltenen $IC_{50}$-Wert (2,82 nM) kann über die entsprechend dem Versuchsansatz vereinfachte Cheng-Prusoff-Gleichung ($K_D$ = $IC_{50}$ (M) - Radioligand (M)) der $K_D$-Wert der Verbindung **[$^{18}$F]JHU94620-D8** gegenüber humanen $CBR_2$-Rezeptoren des Radioliganden berechnet werden: $K_D$ = 2,72 nM. CP55940 ist ein synthetisches Cannabinoid.

Beispiel 7

Bestimmung der $CBR_2$-Gleichgewichts-Dissoziationskonstante $K_i$ von **JHU94620**

Unter Verwendung der Cheng-Prusoff-Gleichung

$$K_i = \frac{IC_{50}}{(1 + \frac{L}{K_D}}$$

wurde die $CBR_2$-Gleichgewichts-Dissoziationskonstante $K_i$ von **JHU94620** bestimmt. L bezeichnet dabei die Konzentration des freien Liganden. Verbindung **JHU94620** wies eine $CBR_2$-Affinität von $K_i$ = 0,4 nM (n = 3) auf. Das Austauschen von Wasserstoffatomen durch Deuteriumionen soll keinen Einfluss auf den $K_i$-Wert haben. Das gilt auch für das Austauschen von Wasserstoffatomen durch Tritiumionen.

## Patentansprüche

1. Verbindung der allgemeinen Formel I

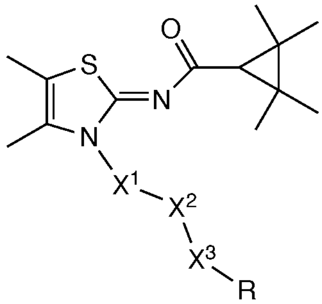

(Formel I),

worin

$X^1$ eine $CD_2$-Gruppe oder eine $CT_2$-Gruppe ist;
$X^2$ Sauerstoff oder eine Gruppe $(CZ^1Z^2)_n$ ist, wobei $Z^1$ und $Z^2$ bei jedem Auftreten unabhängig voneinander jeweils Wasserstoff, Deuterium oder Tritium sind und n eine Ganzzahl von 1 bis 12 ist;
$X^3$ eine $CD_2$-Gruppe oder eine $CT_2$-Gruppe ist; und

R aus der Gruppe ausgewählt ist, die aus Hydroxy, -$NO_2$, Halogen, einem Diazonium-Ion, einem Diazonium-Salz, einem Trialkylammonium-Ion, einem Trialkylammonium-Salz, einem Dialkoxyaren, einem Sulfoxid, einer Boronsäure, einem Boronsäureester, einer zinnorganischen Verbindung, einem Iodonium-Ion, einem Iodonium-Salz, einem Iodonium-Ylid und einem Sulfonat besteht.

**2.** Verbindung nach Anspruch 1, **dadurch gekennzeichnet, dass**

$X^1$ eine $CD_2$-Gruppe oder eine $CT_2$-Gruppe ist;
$X^2$ aus der Gruppe ausgewählt ist, die aus $(CH_2)_n$, $(CD_2)_n$, $(CT_2)_n$ und O besteht, wobei n eine Ganzzahl von 1 bis 12 ist;
$X^3$ eine $CD_2$-Gruppe oder eine $CT_2$-Gruppe ist; und
R aus der Gruppe ausgewählt ist, die aus Hydroxy, -$NO_2$, Halogen, einem Diazonium-Ion, einem Diazonium-Salz, einem Trialkylammonium-Ion, einem Trialkylammonium-Salz, einem Dialkoxyaren, einem Sulfoxid, einer Boronsäure, einem Boronsäureester, einer zinnorganischen Verbindung, einem Iodonium-Ion, einem Iodonium-Salz, einem Iodonium-Ylid und einem Sulfonat besteht.

**3.** Verbindung nach Anspruch 1 oder Anspruch 2, **dadurch gekennzeichnet, dass**

$X^1$ eine $CD_2$-Gruppe ist;
$X^2$ aus der Gruppe ausgewählt ist, die aus $(CH_2)_n$, $(CD_2)_n$ und O besteht, wobei n eine Ganzzahl von 1 bis 12 ist;
$X^3$ eine $CD_2$-Gruppe ist; und
R aus der Gruppe ausgewählt ist, die aus Hydroxy, -$NO_2$, Halogen, einem Diazonium-Ion, einem Diazonium-Salz, einem Trialkylammonium-Ion, einem Trialkylammonium-Salz, einem Dialkoxyaren, einem Sulfoxid, einer Boronsäure, einem Boronsäureester, einer zinnorganischen Verbindung, einem Iodonium-Ion, einem Iodonium-Salz, einem Iodonium-Ylid und einem Sulfonat besteht.

**4.** Verbindung nach einem der vorstehenden Ansprüche, **dadurch gekennzeichnet, dass** n 1, 2, 3, 4, 5 oder 6 ist.

**5.** Verbindung nach einem der vorstehenden Ansprüche, **dadurch gekennzeichnet, dass** R Fluor ist.

**6.** Verbindung nach einem der vorstehenden Ansprüche, **dadurch gekennzeichnet, dass** R [$^{18}$F]Fluor ist.

**7.** Verbindung nach einem der vorstehenden Ansprüche, **dadurch gekennzeichnet, dass** sie

*N*-(3-(4-([$^{18}$F]Fluor)butyl-1,1,4,4-$d_4$)-4,5-dimethylthiazol-2(3*H*)-yliden)-2,2,3,3-tetramethylcyclopropan-1-carboxamid;
*N*-(3-(4-Fluorbutyl-1,1,4,4-$d_4$)-4,5-dimethylthiazol-2(3*H*)-yliden)-2,2,3,3-tetramethylcyclopropan-1-carboxamid;
4-(4,5-Dimethyl-2-((2,2,3,3-tetramethylcyclopropan-1-carbonyl)-imino)thiazol-3(2*H*)-yl)butyl-1,1,4,4-$d_4$-4-methylbenzensulfonat;
*N*-(3-(4-([$^{18}$F]Fluor)butyl-1,1,2,2,3,3,4,4-$d_8$)-4,5-dimethylthiazol-2(3*H*)-yliden)-2,2,3,3-tetramethylcyclopropan-1-carboxamid;
*N*-(3-(4-Fluorbutyl-1,1,2,2,3,3,4,4-$d_8$)-4,5-dimethylthiazol-2(3*H*)-yliden)-2,2,3,3-tetramethylcyclopropan-1-carboxamid; oder
4-(4,5-Dimethyl-2-((2,2,3,3-tetramethylcyclopropan-1-carbonyl)-imino)thiazol-3(2*H*)-yl)butyl-1,1,2,2,3,3,4,4-$d_8$-4-methylbenzensulfonat ist.

**8.** Verbindung nach einem der Ansprüche 1 bis 7 zur Verwendung als Medikament.

**9.** Verbindung nach einem der Ansprüche 1 bis 7 zur Verwendung als Medikament zur Diagnostik und Therapie von Erkrankungen, an denen ein Cannabinoid-Rezeptor 2 beteiligt ist.

**10.** Verbindung nach Anspruch 8 oder Anspruch 9, **dadurch gekennzeichnet, dass** das Medikament ein Radiopharmakon zur nuklearmedizinischen Bildgebung des Cannabinoid-Rezeptors 2 mittels Positronen-Emissions-Tomographie (PET) ist.

**11.** Medikament, enthaltend eine Verbindung nach einem der Ansprüche 1 bis 10 oder ein pharmazeutisch akzeptables Salz davon.

**12.** Verwendung einer Verbindung der allgemeinen Formel I-P

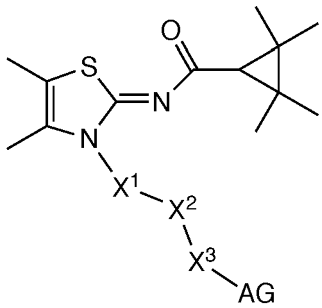

(Formel I-P),

worin

$X^1$ eine $CD_2$-Gruppe oder eine $CT_2$-Gruppe ist;
$X^2$ Sauerstoff oder eine Gruppe $(CZ^1Z^2)_n$ ist, wobei $Z^1$ und $Z^2$ bei jedem Auftreten unabhängig voneinander jeweils Wasserstoff, Deuterium oder Tritium sind und n eine Ganzzahl von 1 bis 12 ist;
$X^3$ eine $CD_2$-Gruppe oder eine $CT_2$-Gruppe ist; und
AG aus der Gruppe ausgewählt ist, die aus Hydroxy, $-NO_2$, Chlor, Brom, Iod, einem Diazonium-Ion, einem Diazonium-Salz, einem Trialkylammonium-Ion, einem Trialkylammonium-Salz, einem Dialkoxyaren, einem Sulfoxid, einer Boronsäure, einem Boronsäureester, einer zinnorganischen Verbindung, einem Iodonium-Ion, einem Iodonium-Salz, einem Iodonium-Ylid und einem Sulfonat besteht,
zur Herstellung einer Verbindung der allgemeinen Formel $[^{18}F]$I-F

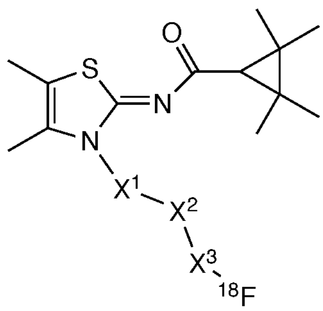

(Formel $[^{18}F]$I-F)

worin $X^1$, $X^2$ und $X^3$ die im Zusammenhang mit der allgemeinen Formel I-P angegebenen Bedeutung haben.

**13.** Verfahren zur Herstellung einer Verbindung der allgemeinen Formel I-F,

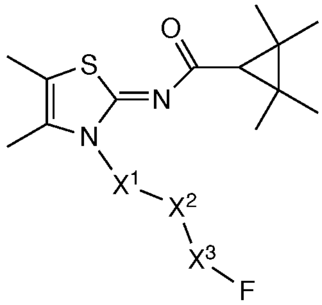

(Formel I-F)

worin

$X^1$ eine $CD_2$-Gruppe oder eine $CT_2$-Gruppe ist;
$X^2$ Sauerstoff oder eine Gruppe $(CZ^1Z^2)_n$ ist, wobei $Z^1$ und $Z^2$ bei jedem Auftreten unabhängig voneinander jeweils Wasserstoff, Deuterium oder Tritium sind und n eine Ganzzahl von 1 bis 12 ist; und
$X^3$ eine $CD_2$-Gruppe oder eine $CT_2$-Gruppe ist;

wobei das Verfahren die Schritte umfasst:

(a) Umsetzen einer Verbindung der Formel 11

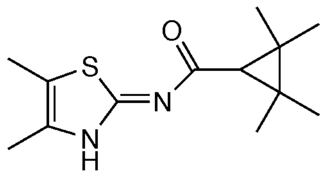

(Formel 11)

mit einer Verbindung der allgemeinen Formel II

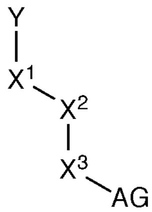

(Formel II)

worin

$X^1$, $X^2$ und $X^3$ die im Zusammenhang mit der allgemeinen Formel I-P angegebenen Bedeutung haben;
Y ein Sulfonat ist; und
AG aus der Gruppe ausgewählt ist, die aus Hydroxy, $-NO_2$, Chlor, Brom, Iod, einem Diazonium-Ion, einem Diazonium-Salz, einem Trialkylammonium-Ion, einem Trialkylammonium-Salz, einem Dialkoxyaren, einem Sulfoxid, einer Boronsäure, einem Boronsäureester, einer zinnorganischen Verbindung, einem Iodonium-Ion, einem Iodonium-Salz, einem Iodonium-Ylid und einem Sulfonat besteht;

zu einer Verbindung der allgemeinen Formel I-P

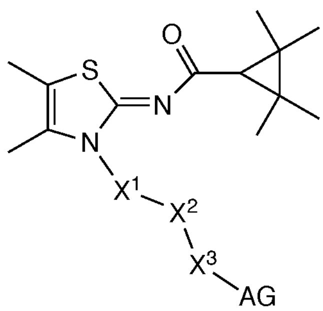

(Formel I-P),

worin $X^1$, $X^2$, $X^3$ und AG die im Zusammenhang mit der allgemeinen Formel II angegebenen Bedeutungen haben; und

(b) Umsetzen der Verbindung der allgemeinen Formel I-P zu der Verbindung der allgemeinen Formel I-F mit

einem Fluorierungsmittel.

**14.** Verfahren nach Anspruch 13, **dadurch gekennzeichnet, dass** Y -O-Ts oder O-Ms ist, wobei Ts eine Tosylgruppe und Ms eine Mesylgruppe bezeichnet.

**15.** Verfahren nach Anspruch 13 oder Anspruch 14, **dadurch gekennzeichnet, dass** Y -O-Ts ist, wobei Ts eine Tosylgruppe bezeichnet.

## Claims

**1.** A compound of general formula I

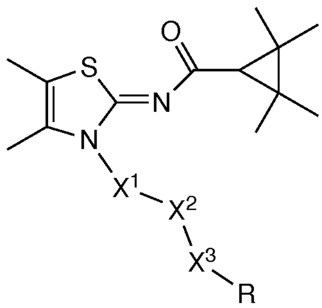

(Formula I),

wherein

$X^1$ is a $CD_2$ group or a $CT_2$ group;
$X^2$ is oxygen or a group $(CZ^1Z^2)_n$, wherein $Z^1$ and $Z^2$ on each occurrence independently each are hydrogen, deuterium, or tritium and n is an integer from 1 to 12;
$X^3$ is a $CD_2$ group or a $CT_2$ group; and
R is selected from the group consisting of hydroxy, $-NO_2$, halogen, a diazonium ion, a diazonium salt, a trialkylammonium ion, a trialkylammonium salt, a dialkoxyarene, a sulphoxide, a boronic acid, a boronic acid ester, an organotin compound, an iodonium ion, an iodonium salt, an iodonium ylide, and a sulphonate.

**2.** The compound according to claim 1, **characterized in that**

$X^1$ is a $CD_2$ group or a $CT_2$ group;
$X^2$ is selected from the group consisting of $(CH_2)_n$, $(CD_2)_n$, $(CT_2)_n$, and O, wherein n is an integer from 1 to 12;
$X^3$ is a $CD_2$ group or a $CT_2$ group; and
R is selected from the group consisting of hydroxy, $-NO_2$, halogen, a diazonium ion, a diazonium salt, a trialkylammonium ion, a trialkylammonium salt, a dialkoxyarene, a sulphoxide, a boronic acid, a boronic acid ester, an organotin compound, an iodonium ion, an iodonium salt, an iodonium ylide, and a sulphonate.

**3.** The compound according to claim 1 or claim 2, **characterized in that**

$X^1$ is a $CD_2$ group;
$X^2$ is selected from the group consisting of $(CH_2)_n$, $(CD_2)_n$, and O, wherein n is an integer from 1 to 12;
$X^3$ is a $CD_2$ group; and
R is selected from the group consisting of hydroxy, $-NO_2$, halogen, a diazonium ion, a diazonium salt, a trialkylammonium ion, a trialkylammonium salt, a dialkoxyarene, a sulphoxide, a boronic acid, a boronic acid ester, an organotin compound, an iodonium ion, an iodonium salt, an iodonium ylide, and a sulphonate.

**4.** The compound according to any of the preceding claims, **characterized in that** n is 1, 2, 3, 4, 5, or 6.

**5.** The compound according to any of the preceding claims, **characterized in that** R is fluorine.

**6.** The compound according to any of the preceding claims, **characterized in that** R is [$^{18}$F]fluorine.

**7.** The compound according to any of the preceding claims, **characterized in that** it is

*N*-(3-(4-([$^{18}$F]fluoro)butyl-1,1,4,4-$d_4$)-4,5-dimethylthiazole-2(3*H*)-yli-dene)-2,2,3,3-tetramethylcyclopropane-1-carboxamide;
*N*-(3-(4-fluorobutyl-1,1,4,4-$d_4$)-4,5-dimethylthiazole-2(3*H*)-ylidene)-2,2,3,3-tetramethylcyclopropane-1-carboxamide;
4-(4,5-dimethyl-2-((2,2,3,3-tetramethylcyclopropane-1-carbonyl)-imino)thiazole-3(2*H*)-yl)butyl-1,1,4,4-$d_4$-4-methylbenzene sulphonate;
*N*-(3-(4-([$^{18}$F]fluoro)butyl-1,1,2,2,3,3,4,4-$d_8$)-4,5-dimethylthiazole-2(3*H*)-ylidene)-2,2,3,3-tetramethylcyclopropane-1-carboxamide;
*N*-(3-(4-fluorobutyl-1,1,2,2,3,3,4,4-$d_8$)-4,5-dimethylthiazole-2(3*H*)-yli-dene)-2,2,3,3-tetramethylcyclopropane-1-carboxamide; or
4-(4,5-dimethyl-2-((2,2,3,3 -tetramethylcyclopropane-1-carbonyl)-imino)thiazole-3(2*H*)-yl)butyl-1,1,2,2,3,3,4,4-$d_8$-4-methylbenzene sulphonate.

**8.** A compound according to any of claims 1 to 7 for use as a medicament.

**9.** A compound according to any of claims 1 to 7 for use as a medicament for the diagnostics and therapy of diseases in which a cannabinoid receptor 2 is involved.

**10.** The compound according to claim 8 or claim 9, **characterized in that** the medicament is a radiopharmaceutical for the nuclear-medical imaging of the cannabinoid receptor 2 by means of positron emission tomography (PET).

**11.** A medicament containing a compound according to any of claims 1 to 10 or a pharmaceutically acceptable salt thereof.

**12.** Use of a compound of general formula I-P

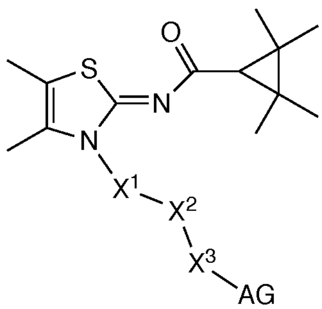

(Formula I-P),

wherein

$X^1$ is a $CD_2$ group or a $CT_2$ group;
$X^2$ is oxygen or a group $(CZ^1Z^2)_n$, wherein $Z^1$ and $Z^2$ on each occurrence independently each are hydrogen, deuterium, or tritium and n is an integer from 1 to 12;
$X^3$ is a $CD_2$ group or a $CT_2$ group; and
AG is selected from the group consisting of hydroxy, $-NO_2$, chlorine, bromine, iodine, a diazonium ion, a diazonium salt, a trialkylammonium ion, a trialkylammonium salt, a dialkoxyarene, a sulfoxide, a boronic acid, a boronic acid ester, an organotin compound, an iodonium ion, an iodonium salt, an iodonium ylide, and a sulphonate,
for the preparation of a compound of general formula [$^{18}$F]I-F

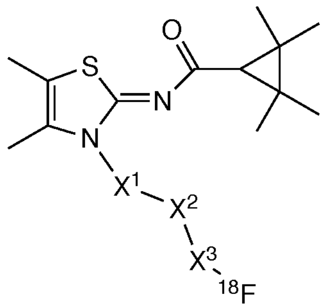

(Formula [18F]I-F)

wherein $X^1$, $X^2$, and $X^3$ have the meanings given in connection with general formula I-P.

**13.** A method for the preparation of a compound of general formula I-F,

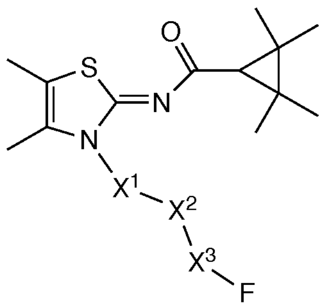

(Formula I-F)

wherein

$X^1$ is a $CD_2$ group or a $CT_2$ group;
$X^2$ is oxygen or a group $(CZ^1Z^2)_n$, wherein $Z^1$ and $Z^2$ on each occurrence independently each are hydrogen, deuterium, or tritium and n is an integer from 1 to 12; and
$X^3$ is a $CD_2$ group or a $CT_2$ group;

wherein the method comprises the steps of:

(a) reacting a compound of formula 11

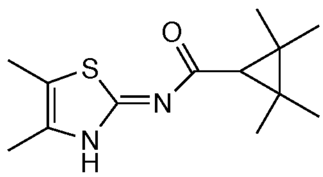

(Formula 11)

with a compound of general formula II

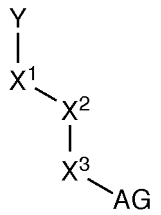

(Formula II)

wherein

$X^1$, $X^2$, and $X^3$ have the meanings given in connection with general formula I-P;
Y is a sulphonate; and
AG is selected from the group consisting of hydroxy, $-NO_2$, chlorine, bromine, iodine, a diazonium ion, a diazonium salt, a trialkylammonium ion, a trialkylammonium salt, a dialkoxyarene, a sulfoxide, a boronic acid, a boronic acid ester, an organotin compound, an iodonium ion, an iodonium salt, an iodonium ylide, and a sulphonate;

to a compound of general formula I-P

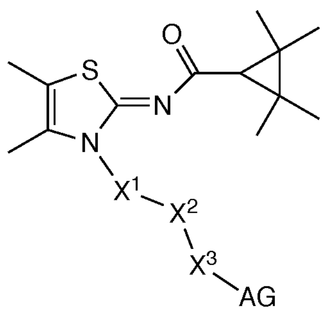

(Formula I-P),

wherein $X^1$, $X^2$, $X^3$, and AG have the meanings given in connection with general formula II; and

(b) reacting the compound of general formula I-P with a fluorinating agent to the compound of general formula I-F.

**14.** The method according to claim 13, **characterized in that** Y is -O-Ts or -O-Ms, wherein Ts designates a tosyl group and Ms a mesyl group.

**15.** The method according to claim 13 or claim 14, **characterized in that** Y is -O-Ts, wherein Ts designates a tosyl group.

## Revendications

**1.** Composé de formule générale I

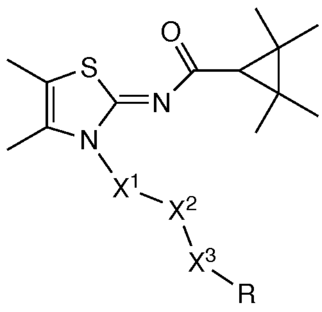

(formule I),

où

$X^1$ est un groupe $CD_2$ ou un groupe $CT_2$ ;
$X^2$ est de l'oxygène ou un groupe $(CZ^1Z^2)_n$ , $Z^1$ et $Z^2$ étant à chaque occurrence indépendamment les uns des autres de l'hydrogène, du deutérium ou du tritium et n étant un nombre entier de 1 à 12 ;
$X^3$ est un groupe $CD_2$ ou un groupe $CT_2$ ; et
R est sélectionné dans le groupe constitué de hydroxy, $-NO_2$, de halogène, d'un ion diazonium, d'un sel de diazonium, d'un ion trialkylammonium, d'un sel de trialkylammonium, d'un dialcoxyarène, d'un sulfoxyde, d'un acide boronique, d'un ester d'acide boronique, d'un composé organostannique, d'un ion iodonium, d'un sel d'iodonium, d'un ylure d'iodonium et d'un sulfonate.

**2.** Composé selon la revendication 1, **caractérisé en ce que**

$X^1$ est un groupe $CD_2$ ou un groupe $CT_2$ ;
$X^2$ est sélectionné parmi le groupe, qui est constitué de $(CH_2)_n$, $(CD_2)_n$, $(CT_2)_n$ et O, n étant un nombre entier de 1 à 12 ;
$X^3$ est un groupe $CD_2$ ou un groupe $CT_2$ ; et
R est sélectionné dans le groupe constitué de hydroxy, $-NO_2$, de halogène, d'un ion diazonium, d'un sel de diazonium, d'un ion trialkylammonium, d'un sel de trialkylammonium, d'un dialcoxyarène, d'un sulfoxyde, d'un acide boronique, d'un ester d'acide boronique, d'un composé organostannique, d'un ion iodonium, d'un sel d'iodonium, d'un ylure d'iodonium et d'un sulfonate.

**3.** Composé selon la revendication 1 ou la revendication 2, **caractérisé en ce que**

$X^1$ est un groupe $CD_2$ ;
$X^2$ est sélectionné parmi le groupe, qui est constitué de $(CH_2)_n$, $(CD_2)_n$ et O, n étant un nombre entier de 1 à 12 ;
$X^3$ est un groupe $CD_2$ ; et
R est sélectionné dans le groupe constitué de hydroxy, $-NO_2$, de halogène, d'un ion diazonium, d'un sel de diazonium, d'un ion trialkylammonium, d'un sel de trialkylammonium, d'un dialcoxyarène, d'un sulfoxyde, d'un acide boronique, d'un ester d'acide boronique, d'un composé organostannique, d'un ion iodonium, d'un sel d'iodonium, d'un ylure d'iodonium et d'un sulfonate.

**4.** Composé selon l'une des revendications précédentes **caractérisé en ce que** n est 1, 2, 3, 4, 5 ou 6.

**5.** Composé selon l'une des revendications précédentes **caractérisé en ce que** R est du fluor.

**6.** Composé selon l'une des revendications précédentes **caractérisé en ce que** R est du $[^{18}F]$fluor.

**7.** Composé selon l'une des revendications précédentes **caractérisé en ce qu'**il est

*N*-(3-(4-($[^{18}F]$fluor)butyle-1,1,4,4-$d_4$)-4,5-diméthylthiazole-2(3*H*)-ylures)-2,2,3,3-tétraméthylcyclopropane-1-carboxamide ;
*N*-(3-(4-fluorbutyle-1,1,4,4-$d_4$)-4,5-diméthylthiazole-2(3*H*)-ylures)-2,2,3,3-tétraméthylcyclopropane-1-carboxamide ;
4-(4,5-diméthyle-2-((2,2,3,3-tétraméthylcyclopropane-1-carbonyle)-imino)thiazole-3(2*H*)-yl)butyle-1,1,4,4-$d_4$-

4-méthylbenzènesulfonate ;
*N*-(3-(4-([$^{18}$F]fluor)butyle-1,1,2,2,3,3,4,4-$d_8$)-4,5-diméthylthiazole-2(3*H*)-ylures)-2,2,3,3-tétraméthylcyclopropane-1-carboxamide;
*N*-(3-(4-fluorbutyle-1,1,2,2,3,3,4,4-$d_8$)-4,5-diméthylthiazole-2(3*H*)-ylures)-2,2,3,3-tétraméthylcyclopropane-1-carboxamide; ou
4-(4,5-diméthyle-2-((2,2,3,3-tétraméthylcyclopropane-1-carbonyle)-imino)thiazole-3(2*H*)-yl)butyle-1,1,2,2,3,3,4,4-$d_8$-4-méthylbenzènesulfonate.

**8.** Composé selon l'une des revendications 1 à 7 pour l'utilisation comme médicament.

**9.** Composé selon l'une des revendications 1 à 7 pour l'utilisation comme médicament à des fins de diagnostic et de traitement de maladies impliquant un récepteur cannabinoïde 2.

**10.** Composé selon la revendication 8 ou revendication 9 **caractérisé en ce que** le médicament est un produit radiopharmaceutique aux fins d'imagerie de médecine nucléaire du récepteur cannabinoïde 2 par tomographie par émission de positons (TEP).

**11.** Médicament contenant un composé selon l'une des revendications 1 à 10 ou un sel de celle-ci, acceptable du point de vue pharmaceutique.

**12.** Utilisation d'un composé de formule générale I-P

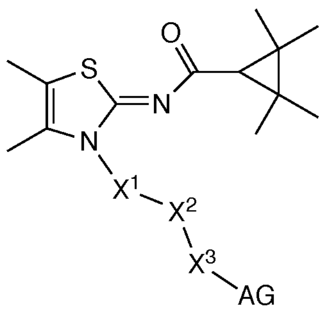

(formule I-P),

où

$X^1$ est un groupe $CD_2$ ou un groupe $CT_2$ ;
$X^2$ est de l'oxygène ou un groupe $(CZ^1Z^2)_n$, $Z^1$ et $Z^2$ étant à chaque occurrence indépendamment les uns des autres de l'hydrogène, du deutérium ou du tritium et n étant un nombre entier de 1 à 12 ;
$X^3$ est un groupe $CD_2$ ou un groupe $CT_2$ ; et
AG est sélectionné dans le groupe constitué de hydroxy, $-NO_2$, de chlore, de brome, d'iode, d'un ion diazonium, d'un sel de diazonium, d'un ion trialkylammonium, d'un sel de trialkylammonium, d'un dialcoxyarène, d'un sulfoxyde, d'un acide boronique, d'un ester d'acide boronique, d'un composé organostannique, d'un ion iodonium, d'un sel d'iodonium, d'un ylure d'iodonium et d'un sulfonate,
pour la préparation d'un composé de formule générale [$^{18}$F]I-F

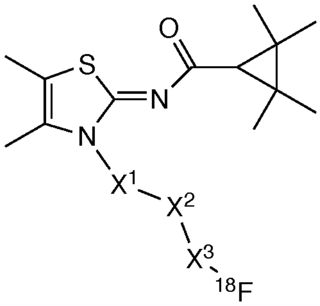

(formule [18F]I-F)

où $X^1$, $X^2$ et $X^3$ ont les significations indiquées en rapport avec la formule générale I-P.

**13.** Procédé pour la préparation d'un composé de formule générale I-F,

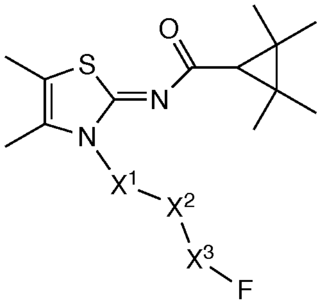

(formule I-F)

où

$X^1$ est un groupe $CD_2$ ou un groupe $CT_2$ ;
$X^2$ est de l'oxygène ou un groupe $(CZ^1Z^2)_n$, $Z^1$ et $Z^2$ étant à chaque occurrence indépendamment les uns des autres de l'hydrogène, du deutérium ou du tritium et n étant un nombre entier de 1 à 12 ; et
$X^3$ est un groupe $CD_2$ ou un groupe $CT_2$ ;

le procédé comprenant les étapes :

(a) Réaction d'un composé de formule 11

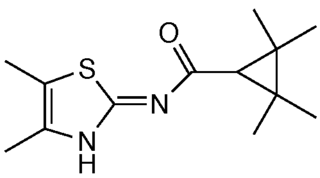

(formule 11)

avec un composé de formule générale II

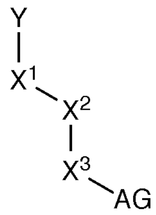

(formule II)

où

$X^1$, $X^2$, $X^3$ ont les significations indiquées en rapport avec la formule générale I-P ;
Y est un sulfonate ; et
AG est sélectionné dans le groupe constitué de hydroxy, $-NO_2$, de chlore, de brome, d'iode, ion diazonium, d'un sel de diazonium, d'un ion trialkylammonium, d'un sel de trialkylammonium, d'un dialcoxyarène, d'un sulfoxyde, d'un acide boronique, d'un ester d'acide boronique, d'un composé organostannique, d'un ion iodonium, d'un sel d'iodonium, d'un ylure d'iodonium et d'un sulfonate ;

en un composé de formule générale I-P

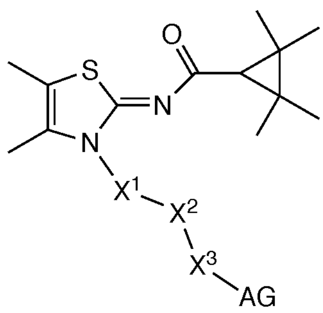

(formule I-P),

où $X^1$, $X^2$, $X^3$ et AG ont les significations indiquées en rapport avec la formule générale II ; et

(b) réaction du composé de formule générale I-P en composé de formule générale I-F avec un agent de fluoration.

**14.** Procédé selon la revendication 13 **caractérisé en ce que** Y est -O-Ts ou O-Ms, Ts désignant un groupe tosyle et Ms un groupe mésyle.

**15.** Procédé selon la revendication 13 ou revendication 14 **caractérisé en ce que** Y est -O-Ts, Ts désignant un groupe tosyle.

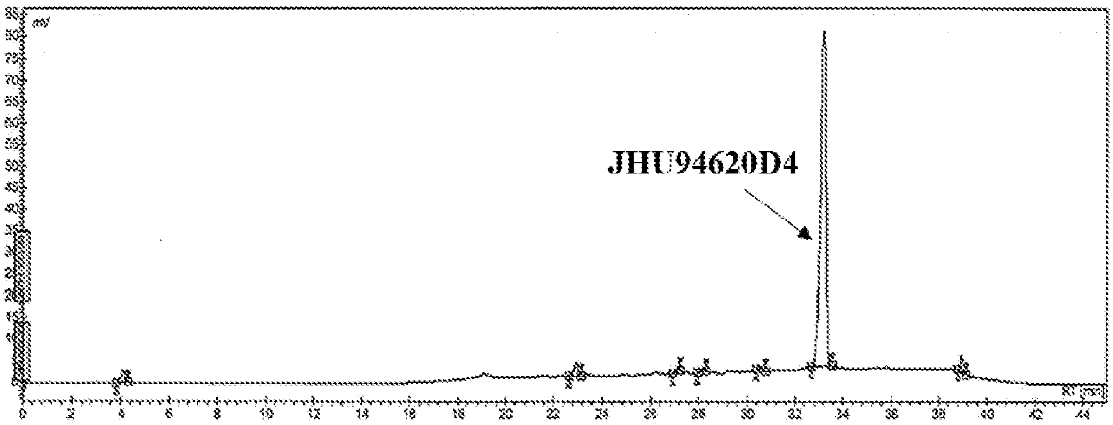


Fig. 1

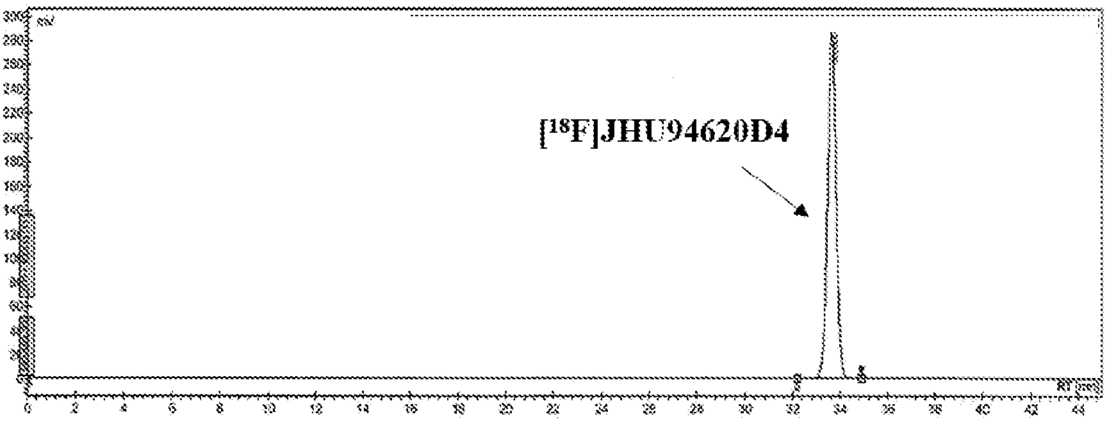


Fig. 2

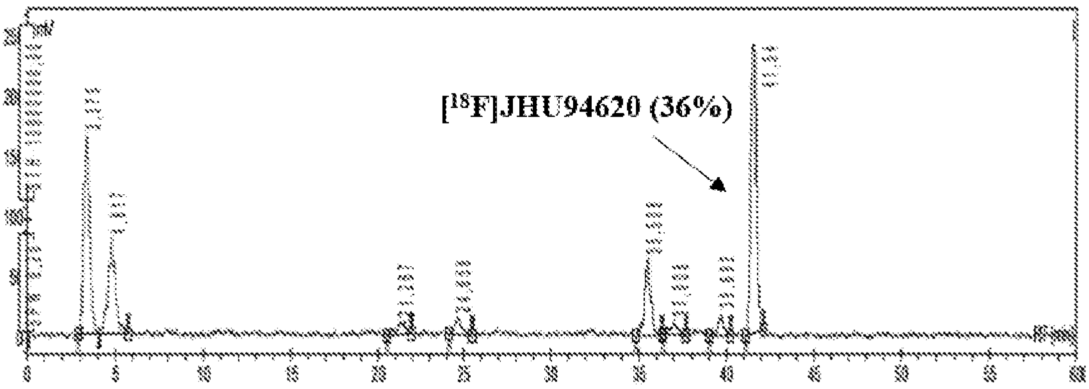


Fig. 3

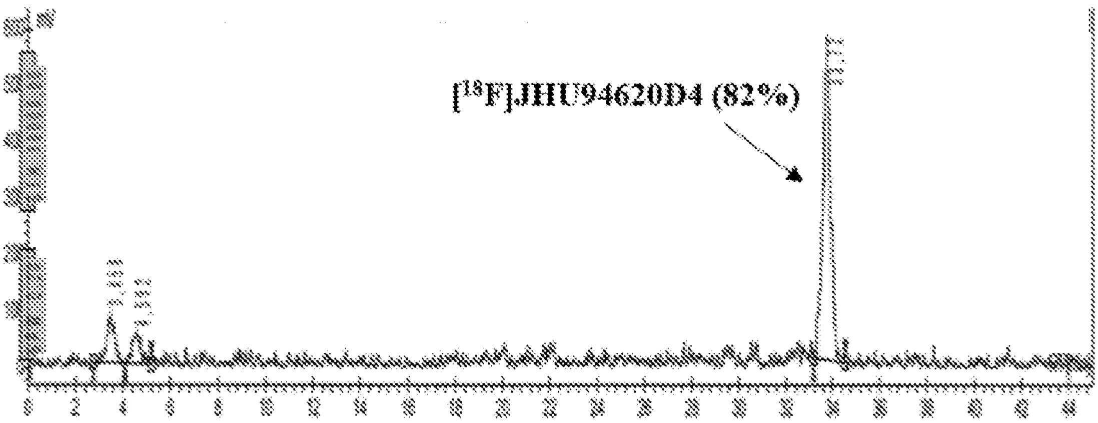


Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009067613 A1


### In der Beschreibung aufgeführte Nicht-Patentliteratur


- **HORTI et al.** *Bioorg. Med. Chem.,* 2010, vol. 14, 5202-5207
- **MOLDOVAN et al.** *J. Med. Chem.,* 2016, vol. 59, 7840-7855
- *J. Nuc. Med.,* 2015, vol. 56 (53), 1048
- **CAILLE et al.** *Mol. Pharmaceutics,* 2017, vol. 11, 4064-4078